(19) **Europäisches Patentamt / European Patent Office / Office européen des brevets**

(11) **EP 2 917 725 B1**

(12) **EUROPEAN PATENT SPECIFICATION**

(45) Date of publication and mention
of the grant of the patent:
**11.07.2018 Bulletin 2018/28**

(21) Application number: **13792947.7**

(22) Date of filing: **12.11.2013**

(51) Int Cl.:
**G01N 27/327** (2006.01)  **G01N 33/543** (2006.01)
**G01N 33/72** (2006.01)

(86) International application number:
**PCT/GB2013/052981**

(87) International publication number:
**WO 2014/072753 (15.05.2014 Gazette 2014/20)**

(54) **PERSONAL TEST DEVICE**

PERSÖNLICHE TESTVORRICHTUNG

DISPOSITIF D'ANALYSE PERSONNEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.11.2012 GB 201220350**

(43) Date of publication of application:
**16.09.2015 Bulletin 2015/38**

(73) Proprietor: **Oxford MEStar Limited**
**Thame**
**Oxfordshire OX9 3EZ (GB)**

(72) Inventors:
• **DILLEEN, John**
**Glasgow**
**Strathclyde G12 8QQ (GB)**

• **HEANEY, Paul**
**Glasgow**
**Strathclyde G12 8QQ (GB)**
• **CHEN, Li**
**Glasgow**
**Strathclyde G12 8QQ (GB)**

(74) Representative: **Lind, Robert**
**Marks & Clerk LLP**
**Fletcher House**
**Heatley Road**
**The Oxford Science Park**
**Oxford OX4 4GE (GB)**

(56) References cited:
**EP-A1- 1 167 968      WO-A1-2010/055306**
**WO-A2-2006/085087    JP-A- H06 201 701**
**US-A- 5 658 531**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

*Field of the Invention*

The present invention relates to a personal test device for analysing a biological sample, particular a faecal sample, comprising an electrochemical sensor. The present invention also relates to methods of taking a biological sample and analysing a biological sample using the personal test device of the invention.

*Background*

Recent developments in healthcare have provided personal test devices for use in a private setting, such as the home of the user. A well-known example of such a device is a home pregnancy test kit. Such are widely available and are well known for their ease of use and reliability at relatively low cost. Many personal test devices are used by healthcare professionals in a healthcare setting to supplement or even replace laboratory-based analyses. Indeed, personal test devices are considered attractive in view of their ability to provide test results quickly using apparatus that is typically handheld. Thus, a healthcare professional may obtain a sample from a subject and test that sample directly, in the presence of the subject. The healthcare professional is then able to provide a diagnosis and even a suitable treatment strategy within a very short time.

The development of further personal test devices has been hampered by the sample preparation step and the analysis. These may be complex.

In particular the analysis of faecal samples is problematic owing to strong personal and cultural aversions to working with such samples. It is desirable to minimise the exposure of the user to faecal matter, even to the extent of minimising the visibility of the sample.

Some disorders of the gastrointestinal (GI) tract are difficult to detect and present detection systems having a camera sensor incorporated into a swallowable pill are often not sufficiently accurate to allow early identification of a problem. Bleeding in the GI tract is a common symptom of several diseases such as Crohn's disease, ulcerative colitis, ulcers and cancer. Bleeding in the GI tract can go unnoticed until it reaches a scale where other symptoms appear, e.g. anaemia, or if fresh blood appears in the stool. By this time, the disease has usually reached an advanced stage. In the case of bowel cancer, polyps often bleed before they become cancerous. Consequently, if they can be detected early, the polyps can be safely removed and the cancer treated successfully. There are known faecal occult blood (FOB) tests, for testing for the presence of blood in stool. These are generally based on the peroxidase-like behaviour of haemoglobin or are based on immunoassays.

One known FOB test uses a guaiac resin impregnated card. Guaiac resin (extracted from trees) changes colour in the presence of oxidising agents. Such tests utilise the fact that haemoglobin catalyses the oxidation of the phenolic compound in guaiac resin (alpha guaiaconic acid) by hydrogen peroxide to form a highly conjugated blue quinone compound. In guaiac-based FOB tests samples of stool are spread by the patient on a card impregnated with guaiac resin. Two samples from each of three stools are typically required to be collected before the card is sent for analysis. In the analysis laboratory, a hydrogen peroxide developer solution is applied to the card and, if blood is present in the sample, a blue-green colour is the result.

The FOB test described above is of use in screening tests, where patients receive the test through the mail, or from their local doctor, take and apply their own samples to the card, and return the card to the laboratory for analysis. The take-up of such tests is variable, particularly amongst the elderly, and amongst people from certain ethnic or social backgrounds, probably due to the unpleasant nature of taking the samples and applying them to the cards.

Recent work in analysing faecal material has provided swallowable capsules that are intended to pass through the lower intestine. Here, *in situ,* the capsule can analyse the intestinal environment and is capable of determining the presence or otherwise of blood at locations that is passes through. The capsule can wirelessly transmit data to a receiver outwith the subject, as the capsule passes through the intestinal tract.

Thus, WO 2006/085087 describes a sensing apparatus, which includes a swallowable pill, having an array of sensor elements where each element is a biological sensor for detecting the presence of the same analyte in the environment in which the sensor array is to be deployed. Activations of a sensor element in the array allows analyte present in the environment of the sensor element, such as haemoglobin, to catalyse a chemical reaction between a first reagent such as alpha gluaiaconic acid, and a second reagent, and the detection of the chemical reaction by the sensor element determines the sensor element output. The first reagent is typically contained within a reagent space of the sensor, which is covered, and may be made available, as required, by removal of that cover to expose the reagent to the local environment.

Whilst a swallowable capsule is capable of providing a detailed analysis of the intestinal tract, its use is not necessarily straight forward. It may be necessary to recover the capsule from a subject's faeces in order to obtain the recorded data, or where wireless data transmission is used during the capsule transit in the intestine, the subject must remain close to a receiver for the length of the examination.

JP 06-201701 illustrates a sampling device for measuring haemoglobin (Hb) in faeces. The device includes a measuring member (13) which includes a faeces gathering sampling member (17) and a developing member (16). Also provided is a container body (2) having pierceable partition walls (7) and (8), which define a developing liquid chamber (5) and a substrate liquid chamber (6). A faecal sample is gathered on the sampling member (17), and this sampling member is inserted into the container body thereby piercing the partition walls (7) and (8) and exposing the faecal sample to substrate from the substrate liquid chamber (6). The developing member (16) receives treated sample from the developing liquid chamber (5) *via* an absorbing pad (14), which is provided on the faeces gathering sampling member (17). Treated sample is drawn up into the developing member (16) by capillary action and Hb in the treated sample is permitted to contact labelled anti-Hb (21), and the resulting immobilised Hb may be detected by e.g. fluorescence. The measuring member (13) is apparently suitable for a single use only, as reuse of the developing member (16) does not appear possible. Therefore, for each reuse of the device, the operator must obtain a further measuring member (13) and a further container body (2). This adds to the overall cost of using this technology for repeated measurements. The design of the sampler is complex, and is not well suited to large scale manufacture.

US 5,658,531 describes a disposable assay device comprising a container body (10) having a reaction chamber (12) sealed with a pierceable membrane (16). The reaction chamber contains an assay reagent (14). The device also includes a sample collector (20) which has a collection chamber (22) and sampling tube (28) connecting with the chamber and for delivery of sample from the collection chamber (22) into the reaction chamber (12). The sampling tube (28) is suitable for piercing the membrane (16). In one embodiment, the container body (10) is provided a multipart membrane (16) having two pierceable membranes (16a) and (16b), which define an internal chamber (17). The internal chamber holds an additional assay reagent (14a) which must be separated from the assay reagent (14). The sampling tube (28) is suitable for piercing the membranes (16a) and (16b) allowing the assay reagents (14) and (14a) to intermix in the presence of sample. The detection of reagents is determined by colorimetric assays, and this requires the use of a transparent reaction chamber (12). The colour change may be detected by eye or by machine. The machine is not a part of the assay device. The disposable assay device is suitable for detecting nicotine metabolites in urine.

A similar assay device to that described in US 5,658,531 is shown in WO 99/38996, WO 2010/129727 and EP 1,167,968.

There is a need to provide a test device for the analysis of biological samples that is genuinely useable in a personal setting.

### Summary of the Invention

In general the present provides personal test devices and methods of using such devices for testing a sample, particularly a biological sample, such as a faecal sample. The devices of the invention allow the sample to be prepared for suitable analysis without unduly exposing the user to chemical or biological reagents. The personal test device avoids the need for the user to have access to test reagents, and avoids the need for sample manipulation prior to analysis. Furthermore, the personal device of the invention incorporates parts that may be reused, thereby reducing the overall cost of the device, which remains an important consideration in the personal device market. Others parts of the device, particularly the part providing the reagents for the analysis, may be replaceable or interchangeable.

Accordingly, in a first aspect of the invention there is provided a personal test device comprising a preparation vessel, a sampler and an analytical unit, wherein:

(i) the preparation vessel has a first chamber with first and second openings, wherein each of the first and second openings is sealed thereby to seal the first chamber, and the first chamber holds one or more reagents;
(ii) the sampler is for holding a sample, and the sampler is adapted to pierce the seals at the first and second openings;
(iii) the analytical unit is adapted to analyse material from the first chamber, and the analytical unit comprises an electrochemical sensor.

In one embodiment, the sampler is for holding a faecal sample. Thus, the personal test device is suitable for the analysis of faecal samples, as shown in the worked examples in the present case.

In one embodiment, the preparation vessel and/or the analytical unit comprises a first reagent and/or a second reagent, the first second reagent being an oxidising agent or a precursor thereof for the first reagent, wherein a reaction between the first reagent and the second reagent is catalysable by an analyte in the sample, such as a protein, to provide a detectable signal. In one embodiment, the first and second reagents are provided in the analytical unit.

In one embodiment, the first and second openings are opposed.

In one embodiment, the preparation vessel and at least a part of the analytical unit are integral. In one embodiment at least a part of the analytical unit is separable from the preparation vessel.

In one embodiment, the preparation vessel and the analytical unit are separate and/or separable parts.

In a second aspect of the invention there is provided a method for analysing a sample, the method comprising the steps of:

(i) providing a test device of the first aspect of the invention;
(ii) obtaining a sample using the sampler of the test device;
(iii) piercing the seal at the first opening of the preparation vessel of the test device with the sampler, and permitting at least part of the sampler to enter the first chamber, thereby to expose sample held by the sampler to the one or more reagents in the first chamber;
(iv) subsequently piercing the seal at the second opening of the preparation vessel with the sampler, thereby to permit material in the first chamber to exit the chamber, such as through the second opening;
(v) permitting the analytical part of the test device to analyse the material from the first chamber.

In one embodiment, the method is for detecting the presence of a blood in a sample.
In one embodiment, the sample is a faecal sample.
In one embodiment, the method is for detecting the presence of haemoglobin in a sample
Described herein is a sampler, such as a faecal sampler, which is suitable for use as a part of the personal test device of the invention, wherein the sampler has a shaft with a piercing tip at one end, and the shaft is provided with one or more protrusions and/or recesses.
The protrusions may be elongate, for example bristles or a comb, extending outwardly from the shaft.
In one embodiment, the shaft is provided with a seal, and the protrusions and/or recesses are located between the piercing tip and the seal. The seal is adapted to seal the first opening.
In one embodiment, the distance between the piercing tip and the seal is greater than the distance between the seals of the first and second openings.
Where the preparation vessel has first and second chambers, the sampler may be provided with first and second seals, where the seals are adapted to seal the first and second openings respectively.
Suitable for use in the personal test is a preparation vessel having a first chamber with first and second openings, wherein each of the first and second openings is sealed thereby to seal the chamber, and the first chamber holds one or more reagents. The seals at the first and second openings are pierceable.
In one embodiment, the preparation vessel is integrated with a part of an analytical unit. Thus, the preparation vessel may have sensing elements.
In one embodiment, the second opening connects the first chamber to a second chamber. The first part of the analytical unit may be provided at the second chamber, thereby to analyse material in the second chamber.
Described herein is a kit comprising one or more of a sampler, a preparation vessel and an analytical unit. The kit may further comprise a set of working instructions. Optionally a pair of gloves, such as surgical style gloves, may be provided with the kit. The kit may comprise a plurality a preparation vessels optionally together with a plurality of samplers for multiple sample measurements.
Other aspects and embodiments of the invention are described in further detail below.

### Description of the Figures

Figure 1 is shows intermediates used in a method for preparing a first part of analytical unit for use in a personal test device according an embodiment of the invention. Figure 1 (f) is the final first part of the analytical unit prepared from the patterned substrate (a) via intermediates (b) to (e).

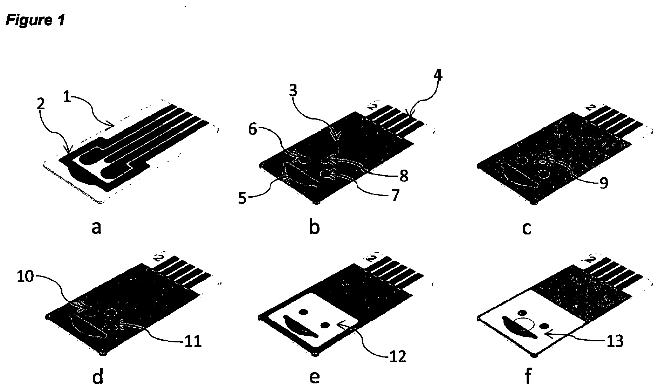

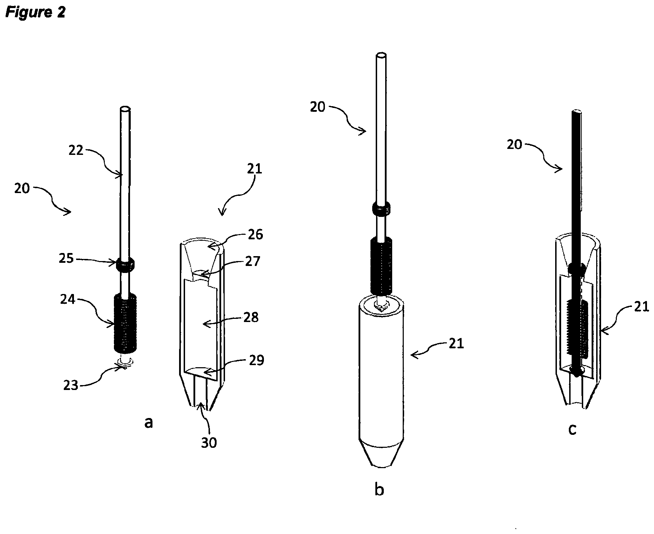

Figure 2 is an illustration of a preparation vessel **21** and a sampler **20** for use in a personal test device according to an embodiment of the invention. The preparation vessel and the sampler are suitable for use together with the first part of the analytical unit of Figure 1 (f). (a) shows the sampler **20** together with a cross section perspective view of a preparation vessel **21** (reagents omitted); (b) shows the sampler **20** poised to enter the preparation vessel **21;** and (c) is a cross section perspective view of the preparation vessel **21** with the sampler **20** inserted into the chamber of the vessel **21,** having pierced the first and second seals.

Figure 3 is an illustration of a personal test device according to a further embodiment of the invention. (a) shows separately a sampler **40,** a preparation vessel and integral first analytical part **42** and a second analytical part **43;** (b) shows the connection of the preparation vessel and first analytical part **42** with the second analytical part **43;** and (c) shows the connection of the sampler **40** with the preparation vessel and integral first analytical part **42** and the second analytical part **43.**

Figure 4 provides cross section views of the personal test device of Figure 3 where (a) is the sampler **40;** (b) is the preparation vessel and integral first analytical part **42;** (c) is the second analytical part **43;** and (d) is the sampler **40** with the preparation vessel and integral first analytical part **42** and the second analytical part **43** (corresponding to a cross section view of Figure 3(c)).

Figure 5 is a an illustration of a preparation vessel and first part of an analytical unit for use in a personal test device according to one embodiment of the invention where (a) is a perspective view of the outer parts of the vessel and integral first analytical part **44;** (b) is a cross section perspective view; and (c) is an alternative cross section view.

Figure 6 is an illustration of a personal test device according to a further embodiment of the invention where (a) shows the personal test device **60** in a perspective view; (b) is side on and plan views of the device (a); (c) is an exploded view of the personal test device (a) showing the sampler **64,** the preparation vessel and first analytical part, and second analytical part **71;** and (d) shows a cross section of sampler **64** with a retracted comb (left hand side) and extended comb (right hand side).

Figure 7 is a graph showing the recorded dose response for test samples having increasing concentrations of haemoglobin in buffer, which have been analysed using an analytical unit including the first part shown in Figure 1(f).

Figure 8 is a graph showing the recorded dose response for blood test samples premixed with buffer and lysing agent, which have been analysed using an analytical unit including the first part shown in Figure 1(f).

Figure 9 is a schematic of the electrochemical set up used in the analytical unit including the first part shown in Figure 1 (f).

Figure 10 is a graph showing the change in current over time for a 0.02 mg/mL Hb solution at the screen printed electrodes of the invention, using a CP/TMB/PER first working electrode and a CP/TMS second working electrode where a + 30 mV bias is applied between the working and reference electrode.

Figure 11 shows the change in recorded charge (nC) with change in Hb concentration within all bran control samples, as measured using a test device as shown in Figure 6.

Figure 12 is a close up of the linear region from Figure 11.

Figure 13 shows the complete study data set for the results shown in Figures 11 and 12, with the test results separated by day.

Figure 14 is a close up is a close up of the low Hb concentration range from Figure 13.

Figure 15 shows the dose response for a data set of spiked ("Hb-dosed") and unspiked ("Raw faeces") faecal samples measured using a test device as shown in Figure 6.

Figure 16 is a cyclic voltammogram showing the current change ($\mu$A) with change in applied voltage (mV) for a selection of spiked and unspiked faecal samples (D35 to D45). Also shown is the response profile for Buffer A (g and h), and Buffer A with Hb (k and l).

Figure 17 shows the dose response curve for a Hb detection method using the HM-Jack test device and the personal test device of Figure 6. The HM-Jack data points are shown as diamonds and the personal test device data points as squares.

### *Detailed Description of the Invention*

The present invention provides a personal test device for analysing a sample, particularly a biological sample. The personal test device of the invention comprises a preparation vessel, a sampler and an analytical unit, which unit has an electrochemical sensor. Described herein is a preparation vessel, a sampler, and an analytical unit, each for use in the personal test device of the invention. Also described herein methods for the use of the preparation vessel, the sampler, and the analytical unit in the analysis of a test sample.

The present inventors have developed a test device which minimises the exposure of a user to the reagents required to prepare a test sample for analysis. The test device provides a complete sample preparation and analysis device, and does so in a device that is portable and may be handheld.

The test device of the invention comprises a preparation vessel that holds the reagents for preparing the sample for analysis. The preparation vessel is sealed to contain the reagents. During the method of analysis the seals of the vessel are broken to allow the reagents to contact the sample, and to allow the resulting material to be provided to an analytical unit for evaluation.

A test device for detecting haemoglobin in a stool sample is described in JP 06-201701, as described in the *Background* section above. The present invention provides several advantages over this known test device. The personal test device described herein has a separate sampler and an analytical unit, allowing the sampler to be disposed of and the analytical unit to be reused. The analytical unit is likely to be the most complex and costly part of the test device, and the ability to reuse at least part of this unit is advantageous.

As described below, and as demonstrated in the worked examples, the personal device is suitable for use with faecal samples, and may be used to determine the haemoglobin content within such a sample.

*Sample*

The present invention provides a personal device for analysing a sample of interest. The sample may be a biological sample. For example, the sample may be a faecal sample, a saliva sample, a hair sample, a blood sample, a urine sample, or a skin sample, amongst others. In one embodiment, the sample is a faecal sample.

The sample may be a liquid sample or a solid sample, or it may be a sample having solids and liquids, for example a fluid, such as a liquid, having solids suspended within it. Intermediate samples of this type may be referred to as semi-solids. A faecal sample is an example of a sample having solid and liquid parts.

In one embodiment the personal test device is for detecting and optionally quantifying the amount of a biological molecule in a sample. That biological molecule may be a polypeptide (protein), polynucleotide, or polysaccharide. Preferably, the biological molecule is a protein, such as a metalloprotein.

In one embodiment the personal test device is for detecting and optionally quantifying the amount of blood in a sample. The test device may be adapted to detect haemoglobin, which is a component of a blood cell.

The present invention is particularly suitable for analysing faecal samples, and may be used to detect blood within that sample, for example by detecting the presence of haemoglobin.

The sample for analysis in the methods described herein is preferably a fresh sample. Thus, the sample is one obtained from a subject (such as a human subject) and is subjected to analysis very shortly afterwards. In one embodiment, the sample is analysed at most 60 minutes, at most 3 minutes, at most 15 minutes, at most 10 minutes, at most 5 minutes, or at most 1 minute after the sample is obtained or deposited from the subject.

The present invention provides a personal device for testing a sample. The user of the device may therefore be the subject from whom the sample is taken. Alternatively, the user of the device may be a carer or health professional responsible for the well-being of the subject from whom the sample is taken.

The sample may be obtained from a subject who is known or is suspected to have a clinically relevant disease or injury. In one embodiment, the subject is one suspected or known to have a disease associated with intestinal bleeding. In one embodiment, the subject is a human having Crohn's disease, ulcerative colitis, ulcers and/or cancer, such as bowel cancer. Here, a faecal sample may be tested. The presence of blood, as indicated by the presence of haemoglobin in the sample, may be an indication of intestinal bleeding.

Where a faecal sample is to be analysed it may be taken from a patient stool. Preferably the faecal sample is taken from the inner portion of the stool, and is not limited to samples taken from the outer surface of the stool. The sampler described herein is provided with a piercing tip and protrusions which allow the sampler to be inserted into a stool to obtain a sample from within. The presence of blood on the surface of the stool sample is not necessarily indicative of a disease as described above. Typically the presence of blood within a core of the stool has a greater association with the diseases described above.

The subject may also be a subject who is undergoing a routine health check. Thus, the methods described herein may be used to identify subjects having a disease or at risk of having a disease. The methods may also be used to verify the well-being of a healthy subject.

It is not necessary for the sample to be modified in any way prior to its use in the methods of the invention. The personal device of the invention is provided with one or more reagents within the chamber of the preparation vessel for the purpose of modifying the sample so that it is in a form suitable for analysis. Thus, it is not necessary for the user of the device to have access to specialised reagents, and nor is it necessary for the user to be required to have experience with sample preparation techniques. All the reagents necessary are provided by the personal test device, and these are exposed to the sample during the method of the invention.

The personal test device of the invention may be used to detect a protein (which may be referred to as an analyte) in a sample.

The protein is capable of catalysing a reaction between a first reagent and a second reagent, which may be an oxidising agent. The first reagent and the oxidising agent may be selected with a target protein (or analyte) in mind. Preferably, the protein is a catalyst for the reaction of hydrogen peroxide with the first reagent.

In one embodiment of the invention, a protein is detected and quantified based on the rate, such as a change in the rate, of the reaction of the first and second reagent. Therefore, in the methods of the invention, the first and second reagents are made available for reaction with the protein at the analytical unit. In certain embodiments, one of the first and second reagents, or both, may be provided in the chamber of the preparation vessel. In other embodiments, one of the first and second reagents, or both, may be provided as a component of the analytical unit, and are made available at an appropriate time for reaction with material from the preparation vessel (for example when the second seal is broken).

The protein may be a metalloprotein Preferably, the protein is horseradish peroxidase or haemoglobin. Most preferably the protein is haemoglobin. In this most preferred embodiment, the device of the invention is suitable for use in the detection of blood within a sample.

The protein may itself be associated with an analyte of interest. The analyte may be a biomolecule, such as those biomolecules that are, or comprise, a polynucleic acid, a polypeptide, or a polysaccharide. The protein may be considered as a label for the analyte that allows the detection, and optionally quantification of the analyte, in a sample. Such detection and analysis is based on the catalytic activity of the protein label.

Typically, the protein is covalently bonded to the analyte, for example by disulfide, amide or ester bonds, as is known to a person of skill in the art. In other embodiments, the protein and the analyte may be linked by other, non-covalent interactions, such as hydrogen bonding.

In the presence of the protein, the first and second reagents react at a rate 100 times or more, 500 times or more, or 1,000 times or more than the reaction of the first and second reagent in the absence of the protein.

The rate of reaction of the first and second reagent in the presence and absence of the protein may be determined electrochemically, as described herein.

*Detection Approach*

In one embodiment, the present invention provides an analytical device for detecting an analyte, such as haemoglobin, in a sample. The analytical unit comprises an electrochemical sensor. The detection method may look to identify and quantify the analyte based on its functional activity. In one embodiment, this activity may be a catalytic activity.

In one embodiment, the analytical device is provided with a reagent whose reactivity is catalysed by the analyte. In one embodiment, the analytical device is provided with a first reagent and a second reagent, and the first second reagent being an oxidising agent or a precursor thereof for the first reagent, wherein a reaction between the first reagent and the second reagent is catalysable by an analyte in the sample, such as a protein, to provide a detectable signal.

The first and second reagents are made available for reaction with components of the sample during the method of analysis.

In one embodiment, at least one of the first and second reagents, such as both of the reagents, may be provided in the analytical unit, to be made available as appropriate, when the analytical unit analyses the mixture from the chamber. In one embodiment, at least one of the first and second reagents, such as both of the reagents, may be provided in the first chamber of the preparation vessel unit, to be made available when the sample is provided into the chamber. The resulting mixture, which is permitted to exit the chamber, through the second opening, may then be analysed by the analytical unit.

The first reagent is reactable with the second reagent in the presence of the analyte, such as a protein. Preferably the second agent is hydrogen peroxide, or a precursor thereof. Therefore, the first reagent is preferably a compound that reacts with hydrogen peroxide in the presence of the protein.

Preferably the first reagent is, or comprises, a compound selected from tetramethylbenzidine, alpha guaiaconic acid, 2,2'-azino-bis(3-ethylbenzothiazolidine-6-sulphonic acid), hydroquinone, phenylenediamine, o-dianisidine, o-tolidine (dimethylbenzidine), 6-methoxyquinoline, and 3,3'-diaminobenzidine, 3-amino-9-ethylcarbazole.

Preferably the first reagent is, or comprises, tetramethylbenzidine.
Preferably the first reagent is, or comprises, 3,3',5,5'-tetramethylbenzidine.

In the most preferred embodiment, the first reagent is, or comprises, tetramethylbenzidine. This reagent is particularly suitable for use in the invention as it is not believed to be harmful to human subjects.

The second reagent is an oxidising agent or a precursor thereof. It is reactable with the first reagent in the presence of the protein.

Preferably the second reagent comprises hydrogen peroxide or a precursor thereof. Preferably hydrogen peroxide is releasable from the second reagent upon contact of the second reagent with water.

The hydrogen peroxide is reactable with the first reagent in the presence of the protein.

Preferably the second reagent is, or comprises, a compound selected from urea peroxide, a perborate compound and a periodate compound. In one embodiment, the second reagent is, or comprises, urea peroxide. In another embodiment, the second reagent is, or comprises, a perborate compound, preferably sodium perborate. For *ex vivo* applications, sodium perborate is preferred as the resulting electrode has a greater stability than those electrodes comprising other reagents, for example those electrodes comprising urea peroxide. In particular, those electrodes comprising sodium perborate have been found to have a longer shelf-life.

*Preparation Vessel*

The preparation vessel is provided with a sealed chamber. The chamber holds one or more reagents for mixing with a sample. The chamber has a first opening and a second opening. These openings are sealed. In use the seals are broken allowing sample to be added to the chamber, and allowing a mixture of the sample and the reagents to exit from the chamber. The preparation vessel holds the reagents until they are required for analysis. Thus, the preparation vessel may be conveniently stored and transported. The preparation vessel is intended to provide all the reagents necessary for preparing the sample for analysis. Thus, it is not necessary for the user of the device to have access to other chemicals in order to perform a sample analysis. The user of the device is not unduly exposed to the reagents, as these are contained in the vessel until they are needed. In some embodiments, the reagents are not released from the vessel and remain within the vessel for the analysis steps. After analysis is complete, the preparation vessel may be disposed of.

The preparation vessel is provided with a first opening which is a fluid passage from outside the vessel through a vessel wall to the chamber. The first opening is sealed with a first seal. The first seal prevents material passing through the first opening. In use the first seal may be broken by a sampler, as described herein. In use, at least part of the sampler is permitted to pass through the first opening and enter the chamber. The first opening may be referred to as a passage for a part of the sampler.

The first seal may be provided at the first opening substantially flush with the exterior surface of the preparation vessel. Thus, the seal must be broken before any part of the sampler can pass through the opening.

The first seal may be provided within the opening, for example the first seal may be provided at the first opening substantially flush with an interior surface of the preparation vessel i.e. the chamber wall. Alternatively, the seal may be placed at a location in the first opening that is between the inner and outer surfaces of the preparation vessel Thus, a part of the sampler can pass through at least part of the opening before it is necessary to break the seal. This arrangement is advantageous as the piercing tip of the sampler may be contained within the first opening. If the user has problems piercing the seal, the piercing tip is likely to remain within the opening during the piercing process. Where the seal is located at the first opening substantially flush with the exterior surface, there is a risk that the piercing tip may skew off the surface, with the risk that it may contact the user's hand. In other embodiments of the invention, the preparation vessel may be contained within a larger housing, which is adapted to also hold part of the sampler. The housing may contain the piercing tip, thereby to prevent the tip from contacting the user's hand during a failed piercing attempt.

The cross-section shape of the first and second openings may be matched with the general cross section shape of the sampler. For example, the first and second openings may be sustainably circular in cross section where the shaft of the sampler has a generally circular cross section.

In one embodiment, the first opening is tapered along its length. The opening tapers towards the chamber from the outer vessel wall. In this embodiment, the opening may guide the sampler to the seal, which may be located within the opening (for example, the seal is not flush with the exterior surface of the preparation vessel).

The preparation vessel is provided with a second opening. In one embodiment the second opening is a fluid passage from the chamber through the vessel wall. In one embodiment the second opening may be a fluid passage from the chamber through a vessel wall to the outside of the vessel. In an alternative embodiment, the second opening may be a fluid passage from the chamber (the first chamber) to a second chamber in the vessel.
The second opening is sealed with a second seal. The second seal prevents material passing through the second opening. In use the second seal may be broken by the sampler. In use, at least part of the sampler is permitted to pass from the chamber into the second opening. The second opening may be referred to as a passage for a part of the sampler. As with the first seal, the second seal may be placed at any location within the second opening.

In use the first opening is held above the second opening. Thus, once the second opening is pierced, material in the chamber will pass from the chamber through the second opening. The preparation vessel may be suitably marked to indicate to the user which way the preparation vessel should be held in use. However, in some embodiments the preparation vessel may be substantially symmetrical and the first and second opening may be indistinguishable.

In use, material may be prevented from exiting the chamber via the first opening. For example, the sampler for use with the preparation vessel, once inserted into the preparation vessel, may block the first opening, thereby to limit or prevent material from exiting the chamber through this opening. As described herein, the sampler may be provided with a seal specifically for this purpose.

In one embodiment the first and second openings are aligned, or opposed, across the chamber. Thus, the sampler may pass through the first opening and may be guided by the walls of the opening across the channel to the second opening. In this way it is a relatively simple task to pierce the second seal, even though this action cannot necessarily be viewed by the user.

As noted above, the second opening may be a fluid passage through the vessel wall connecting the chamber to the outside of the vessel. After the second seal is pierced material from within the chamber is permitted to exit the chamber through the second opening, leave the preparation vessel and pass on to the analytical unit for analysis. In a simple embodiment of the invention the material passing through the second opening is allowed to drop from the preparation vessel onto the analytical unit, such as a first part of the analytical unit. Thus, the user simply needs to place the second opening above the appropriate part of the analytical unit during this stage of the analysis procedure.

In an alternative arrangement, the second opening may be a fluid passage through a vessel wall connecting the chamber (the first chamber) to a second chamber. After the second seal is pierced, material from within the first chamber is permitted to exit the chamber through the second opening, and pass to the second chamber where it is available to at least part of the analytical unit for evaluation.

The chamber may be referred to as the first chamber. The second opening of the first chamber, once the second seal is pierced, may provide a fluid communication to the second chamber. Where a second chamber it provided it may be in fluid communication only with the first chamber via the second opening. When the second opening is sealed, the second chamber is effectively sealed from the contents of the first chamber, and also from the outside environment. Consequently, the second chamber may be considered as a sealed environment, and may also be an anhydrous environment and/or an anaerobic environment. Advantageously, the part of an analytical unit which is present to evaluate material in the second chamber may be maintained in this protected atmosphere until it is needed i.e. when the second seal is pierced and the material from the first chamber is permitted to enter the second chamber. Thus, the part of the analytical unit may be prevented from deteriorating. The change in the environment of the second chamber, once the second seal is broken, may be detected by the sensor. This change may be a useful start point for the analysis, as it is a useful indicator of when the sample has contacted the reagents held within the first chamber.

The preparation vessel holds one or more reagents, as described below. In use, the reagents mix with a sample that is introduced into the chamber via the first opening, and the resulting mixture is permitted to exit the chamber via the second opening.

The shape and dimensions of the preparation vessel are not particularly limited. However, the present invention relates to personal devices, therefore the vessel is adapted to be hand held.

The largest dimension of the preparation vessel may be at most 30 cm, at most 20 cm, at most 10 cm. Thus, the preparation vessel is of a size to be held in one hand. The largest dimension of the preparation vessel may be at least 1 cm, at least 5 cm or at least 10 cm. It is important that the vessel is not too small in size to make it hard to manipulate by the user, who may lack dexterity (e.g. as is common amongst elderly or infirm users).

The preparation vessel may be roughly cylindrical or cuboidal in shape. Such vessels are easily grasped. One or more protrusions and/or recesses may be provided on the outer walls of the preparation vessel to aid the grip of the vessel by the user. This may be important as a firm hold of the preparation vessel may be required during the piercing steps of the first and second seals.

In one embodiment, the vessel may be provided with a window for viewing the contents of the chamber. The window may be provided to view the second opening and the second seal. Where a second chamber is provided in the preparation vessel, a window may be provided to view the contents of the second chamber.

US 5,658,531 describes an assay device comprising a container body having a reaction chamber. In the preferred container bodies, the reaction chamber is sealed with a single pierceable membrane. In one alternative embodiment, the reaction chamber is provided with two pierceable membranes. The purpose of these membranes is to separate reagents within the reaction chamber. In contrast, the arrangement of first and second seals in the preparation vessel of the present invention is to separate the analytical unit from the first reaction chamber. Once the second seal is pierced, material from the first reaction chamber is permitted to exit the chamber, and that material is analysed by the analytical unit.

Vessel Reagents

The preparation vessel is provided with a chamber holding one or more reagents. The reagents are provided for mixing with the sample in the chamber. The resulting mixture of sample and reagents is allowed to exit the chamber via the second opening to be evaluated by the analytical unit.

The one or more reagents may be provided in order to prepare the sample for analysis. For example, the reagents may dilute the sample, may dissolve components, such as an analyte of interest, in the sample, and may precipitate components.

In one embodiment, the chamber holds water, optionally together with other reagents. The chamber may hold an aqueous mixture at a pH in the range pH 1-12, for example pH 3-11, for example pH 5-9. The aqueous mixture may be buffered to maintain the mixture at a particular pH, or a range of pHs.

Reagents may also be used to convert an analyte of interest into a form suitable for evaluation.

In one embodiment, the reagents are for stabilising an analyte of interest. Where the sample comprises a biological molecule, such as a protein, the chamber may be provided with reagents that are together a buffer for the biological molecule. The buffer may be provided to maintain the biological molecule in solution. The buffer may be provided to maintain the structural and functional activity of the biological molecule, for example the molecule's ability to catalyse the reaction of a first reagent with a second reagent, as described herein.

In one embodiment, the chamber holds a citrate-phosphate buffer.

The analytical unit has an electrochemical sensor for the analysis of the mixture from the preparation vessel. Here, the mixture from the reaction vessel is an aqueous electrolyte comprising an analyte of interest. Thus, the reagents in the chamber may make up an electrolyte.

In one embodiment of the invention, the chamber holds a cell lysing agent. The agent is suitable for reaction with a cell in the sample, thereby to release the cell contents.

In one embodiment, the cell lysing agent is saponin.

In one embodiment, the chamber holds an inorganic salt, such as KCl or NaCl.

In one embodiment, the chamber may contain a wetting additive. Such may be used together with an electrochemical sensor within the analytical unit, to accelerate the hydration of the electrode, thereby to provide accelerated response times. Additionally or alternatively, the wetting agent may be provided in a working electrode.

In one embodiment, the chamber holds the first and/or second reagents that are described herein. In one embodiment, the second chamber, where present, holds the first and/or second reagents. Preferably, however, the first and second reagents are provided as components of the analytical unit.

As described herein, in one embodiment, the invention provides a method of detecting an analyte, such as a protein, where that analyte is a catalyst for the reaction of a reagent, such as a first reagent with a second reagent. In one embodiment, the reagents for this reaction are not provided in the chamber, and are provided outwith the chamber, for example as part of the analytical unit. Here, the reagents that are provided in the chamber may be used to prepare the sample for the catalytic reaction that is monitored in the analytical unit. For example, the purpose of the chamber reagents may to provide the analyte of interest available for reaction. Where the sample for analysis contains cells such as blood cells, the reagents in the chamber may be used to lyse the cells in order to make the cell contents available.

Manufacture of Preparation Vessel

A preparation vessel may be prepared from a suitable vessel having suitable first and second openings that provide fluid passage to an internal chamber. In general one of the openings is sealed, and one or more reagents is added to the chamber. The remaining opening is then sealed thereby to provide a preparation vessel for use in the methods described herein.

Typically, the walls of the preparation vessel that define the chambers and the openings are of a strong polymeric material. Example materials include high density polyethylene (HDPE), such as Exxon HDPE HMA016, polypropylene (PP) or acrylonitrile butadiene styrene (ABS). The walls of the preparation vessel may be prepared by suitable molding techniques familiar to those of skill in the art.

Typically, the walls of the vessel are formed, and then a seal at the first or second opening is added. The reagents are added to the first chamber, and then the other of the first or second opening is added. Where the vessel has a second chamber, it is preferred that the second opening is sealed first, and the reagents are added to the first chamber via the first opening. The first opening is then sealed, to seal the first chamber.

The seals in the first and second openings may be any material that is strong enough to contain the reagents in the first chamber, yet is capable of being pierced by a user operating the sampler. An example material for use is aluminium foil, such as Alcan foil. The seals may be made by heat sealing the aluminium foil to the walls of the preparation vessel at or in the first and second openings. The seal may be heat sealed to the material to the walls of the preparation vessel.

The material of the vessel walls and the seals is strong enough to contain the reagents within the chamber during storage. Thus, the walls and the seals must have structural strength and must be chemically inert to the reagents.

Where the vessel is integrated with a first part of the analytical unit, this first part may form part of the wall of the vessel at the second chamber. The first part may be inserted into a suitable opening provided in the vessel at the second chamber, and may be fixed there.

*Sampler*

The personal test device is provided with a sampler for holding a sample. The sampler is for use together with the preparation vessel and is adapted to pierce the seals at the first and second openings of the preparation vessel. The sampler is suitable for holding a sample and, in use, delivers the sample into the chamber of the preparation vessel, where the sample is exposed to the reagents held within. The sampler and the analytical unit are not integrated, and are separable units with the personal test device.

Generally, the sampler has a piercing tip to pierce the seals of the preparation vessel. In one embodiment, the piercing tip, in use, provides a hole in the first and second seals that is substantially the same size and/or shape as the piercing tip.

The shape of the piercing tip is not particularly limited, and the size and shape of the tip may be adapted for use with particular seals. Generally, the piercing tip is tapered to a point, which is suitable for providing the hole in a seal.

The piercing tip may be integral with the shaft.

In one embodiment, the sampler is suitable for holding a solid sample.
In one embodiment, the sampler is suitable for holding a faecal sample.

The sampler, such as a faecal sampler, is a part of the personal test device of the invention, wherein the sampler has a shaft with a piercing tip at one end, and protrusions and/or recesses provided on the shaft. In use, the protrusions and/or recesses are suitable for holding sample. Thus, sample may be held on or between the protrusions. Sample may be held in recesses. Preferably, the shaft has one or more protrusions.

The piercing tip and the protrusions, where present, allow the sampler to be inserted into, for example, a stool from a subject. In this way a faecal sample may be taken from an inner portion of the stool.
The protrusions may take any suitable form. The protrusions may be in the form of elongate protrusions, which may be flexible or inflexible. A collection of flexible elongate protrusions may be referred to as bristles. A collection of inflexible elongate protrusions may be referred to as a comb.
The protrusions may be provided on the shaft spaced from the piercing tip at a shaft end. The protrusions may be integral with the shaft.

In one embodiment the sampler has a shaft with a piercing tip at one end, and flexible bristles extending outwardly from the shaft.

In one embodiment, the shaft is provided with a seal, and the protrusions are located between the piercing tip and the seal. The seal may be spaced apart from the protrusions on the shaft.
The seal is adapted to seal the first opening. In use the shaft of the sampler passes through the first opening. The seal is larger than the first opening, and when the seal abuts the preparation vessel at the first opening, the shaft is prevented from moving further through the opening. With the seal abutting the first opening, material is prevented from exiting the chamber through the first opening. Thus material passes out of the chamber from the second opening only.
In one embodiment, the distance between the piercing tip and the seal is greater than the distance between the seals of the first and second openings.
The seals provided on the sampler shaft may be O-ring seals.

The shaft may be generally cylindrical in shape. Alternatively, the shaft may be generally cuboidal in shape.

The protrusions on the shaft extend from the shaft in a direction radial to the longitudinal axis of the shaft. The protrusions may extend from the shaft for a distance that is greater than the distance the piercing tip extends in a radial distance from the shaft. This is preferred where the protrusions are flexible. This arrangement is particularly useful as the protrusions will flex as they pass through the seal piercing - the piercing in the seal may have cross section dimensions that are less than the cross section dimension of the sampler at the location of the protrusions, thus it may be necessary for the protrusion to flex to allow the sampler to pass through the piercing. As the sampler continues to enter the preparation vessel, the protrusions flex back to their original position. Sample that is held on or between the protrusions may be flicked from the protrusions as they flex back, allowing the sample to be dispersed into the reagents within the chamber.

Alternatively, the protrusions may extend from the shaft for a distance that is equal to or less than the distance the piercing tip extends in a radial distance from the shaft. This is preferred where the protrusions are inflexible.

The sampler may be provided with a handle, which may be connected at the shaft at the end portion of the shaft distal to the piercing tip. The handle may be provided to aid the manipulation of the sampler, where it is inconvenient to hold the shaft directly.

In one embodiment, the shaft is retractable into the handle, for example to retract the piercing tip into the handle, thereby containing the piercing tip and preventing it from injuring the user. In use, the shaft may be extended from the handle.
In another embodiment, the handle comprises a sheath, which may be extended from the handle to at least partially cover the shaft, the protrusions and optionally also the piercing tip. In use, the sheath is removable, for example it may be retracted to expose the shaft, the protrusions and the piercing tip.

The shaft, piercing tip and protrusions may be formed from a polymeric material, such as those materials for use in the preparation of the walls of the preparation vessel. The shaft, piercing tip and protrusions may be molded in one piece. A handle, where present, may be a one piece with the shaft, piercing tip and protrusions. Alternatively, the handle may be added to the end of the shaft distal to the piercing tip, after the shaft has been formed. Where the sampler has one or two seal, these may be placed on the shaft prior to the addition of the handle.

The sampler described in JP 06-201701 is a smooth shaft. It may not be particularly well suited to retaining faecal matter on its surface. The provision of protrusions and/or recesses on the present sampler allows for increased sample retention. Moreover, flexible bristles may also be sued to increase the distribution of faecal matter in the preparation vessel, by the flexing of those bristles as they pass through the first opening. Similarly the sample collector of US 5,658,531 is primarily designed for liquid samples and would not be expected to retain faecal matter. The protrusions and/or recesses that may be provided on the present sampler allow liquids and solid matter to be held and delivered to the chamber of the preparation vessel.

*Analytical Unit*

The present invention provides an analytical unit for analysing the mixture from the first chamber of the preparation vessel. The analytical unit comprises an electrochemical sensor. The reagents that are provided in the first chamber are for preparing a sample so that it is in a form suitable for evaluation by the analytical unit. The analytical unit may be adapted to analyse material that exits from the first chamber of the preparation vessel.

As noted above, the sampler and the analytical unit are not integrated, and are separable units with the personal test device. The present case allows the analytical unit to be retained after use, for use in subsequent analyses. The sampler is a disposable article, and it may be readily replaced with another sampler for a second analysis. This avoids the need to clean the sampler for reuse in subsequent analyses. JP 06-201701 describes a test device where the sampler and analytical unit are integrated. After a single analysis it appears that the combined sampler and analytical unit must be disposed of and this adds to the cost of the device. Alternatively, both parts must be carefully cleaned to allow their reuse. This is particularly difficult for a device intended to be used within the home and amongst patient populations for whom careful cleaning may present a challenge (e.g. the elderly).

The analytical unit may comprise detecting elements for making an analysis of the mixture from the first chamber. The detecting elements may be exposable to the mixture from the first chamber. In use, the sensing elements may contact the mixture.

The analytical unit also comprises a controller to control the detecting elements, and a power supply for the controller and the detecting elements. The analytical unit optionally further comprises a display to provide the user with a visual indication of the analysis results. The analytical part may also include means for transferring recorded data to another device.

The personal test device of the invention is a hand-held device. Thus, the dimensions of the device are such that allow the personal device to be easily held and manipulated by the user. It follows that the analytical unit is also sufficiently small to allow its incorporation into the personal test device.

The analytical unit may comprise a first part and a second part, which parts may be connected or are connectable (i.e. they may be separable). The first analytical part may comprise the detecting elements. The first analytical unit may be disposable, and may be replaced in a personal test device after use with another first analytical unit. Thus, the first analytical unit may be a one-shot product.

Typically the second part comprises the controller and the power unit, optionally together with the display and/or the means for transferring recorded data. The second analytical unit may be reusable and for repeat analyses may be used together with replacement first analytical parts. The ability to reuse at least part of the analytical unit, such as the second analytical part, therefore minimises costs for the use of the personal test device.

The first analytical part may be integrated with the preparation vessel. For example, the first part may be provided in a vessel to analyse material that exits the first chamber. In one embodiment of the invention, the preparation vessel is provided with a second chamber which is connected to the first chamber by a second opening. The first analytical part may be provided at the second chamber to analyse material that passes into that chamber from the first chamber *via* the second opening. The second opening is sealed to prevent the reagents in the first chamber from entering the second chamber. In use, the sampler pierces the second seal to permit material to move from the first chamber into the second. The sensing elements of the first analytical part may be provided within a wall of the vessel, exposed to the second chamber. The sensing elements are connected or connectable to the second part of the analytical unit. This connection may be in the form of an electrical connection.

The analytical unit may comprises a sensor device based on electrochemical detection.

The analytical unit comprises an electrochemical sensor for performing an electrochemical analysis of the sample. The electrodes of the sensor may be provided as the sensing elements in the first part of the analytical unit.

In one embodiment, the analytical unit is an electrochemical sensor having a working electrode, a counter electrode, a power supply and a controller. These parts are described in further detail below. In one embodiment, the working electrode and counter electrode are connectable to a power supply and a controller, and the electrodes are provided in a first part analytical part and the power supply and the controller are provided in a second analytical part, which is connectable to the first part and is in electrically communicable with the first analytical part.

The analytical unit may be provided with a display for showing the results of an analysis. The display may also be suitable for providing instructions for the use of the device in a method of analysis. The display may take the form of a series of a lighting system for displaying different colours to the user in view of different analysis results. Alternatively or additionally, the display may be capable of displaying characters, for example to provide instructions to the user, or to display the results in written form, optionally together with instructions for the user in the light of the test results, for example to seek the advice of a physician.

The power supply of the analytical unit may be provided by batteries contained within the unit. These batteries may be rechargeable, and the analytical unit may be provided with suitable means, such as electrical contacts for recharging the batteries *in situ.* Alternatively, the batteries may be removable for recharging, or are removable to be replaced with new batteries.

The personal test device of the invention may be powered by direct connection to the mains electricity supply. However, this is not preferred for a personal test device, which is ideally easily moveable, and is useable in environments where the electricity supply is not readily available, e.g. within a bathroom, or simply by geographical locations e.g. within the developing world.

Electrochemical Sensor

In one embodiment the analytical unit comprises an electrochemical sensor having a working electrode and a counter electrode, optionally together with a reference electrode. The electrodes may together be referred to as sensing elements. The electrochemical sensor may also comprise a power supply, a controller, and a current detector.

The present inventors have found that an electrochemical sensor may be readily incorporated into a personal test device, as the components of the sensor are suitable for miniaturisation, are generally robust, and not have a particularly high power demand.

The test devices described in JP 06-201701, US 5,658,531, WO 99/38996, WO 2010/129727 and EP 1,167,968 do not incorporate an electrochemical sensor. These documents do not show how such a sensor could be used within a personal test device.

The reagents in the chamber of the preparation vessel may be contained, such as dissolved, in water. Together these components may make up an electrolyte for electrochemical measurements. Once the second seal is pierced, these components may flow out of the chamber, and may come into contact with the electrodes of the electrochemical assembly.

As noted above, a part of the analytical unit may be contained within a sealed second chamber which is accessible from the first chamber via the second opening in the first chamber. It will be appreciated that the electrodes may be provided in the sealed second chamber, and subsequently they maintained in a substantially anhydrous environment until the second seal is pierced, and reagents and sample are permitted to move from the first chamber into the second chamber.

Maintaining the electrodes in an anhydrous form is advantageous. The initial wetting of the electrodes may provide a simple start point for the timing of an electrochemical reaction. The electrodes for use in the present invention include electrodes having reagents contained on or within the electrode. Maintaining the electrodes in anhydrous form prevents those reagents from leaching of the electrode and away from the electrode work surface. Once the electrodes are exposed to the electrolyte, the reagents are permitted to leach from the electrode, where they will be available at the electrode work surface to participate in the relevant chemistry.

The present inventors have previously described the preparation and use of carbon paste working electrodes comprising one or more reagents for use in the detection of a protein in a sample (see WO 2009/055306).

The present invention provides a vessel having a first part of an analytical unit, and a separate second part of an analytical system, which is connectable to the first part. The electrodes may be adapted to electrically connect to the second part. For example, the electrode may be provided with suitable electrically conducting contacts for connection to corresponding electrical contacts on the second part.

In one embodiment, the electrodes may be provided in the vessel, where the electrode work surfaces are exposed to the second chamber. The electrode contacts may pass through the vessel wall to the outside surface of the vessel, where they are available for connection to the second part

The second part of the analytical unit may comprise the power supply, controller, and current detector. These parts are electrically connectable to the first analytical part.

The first analytical part may comprise a sheet, including a disc, having electrodes on one face. The electrical contacts for those electrodes may extend from the electrode to the edges of the sheet, where they may be available for electrical connection. In other embodiments, the electrical contacts for the electrodes extend through the sheet (for example is prepared openings in the sheet) where they are made available for electrical connection at the other face of the sheet. This latter arrangement is of use in the embodiments where the electrochemical sensor is integral with the preparation vessel, and the electrode surfaces are available for contacting material in the second chamber. Contacts are provided through a vessel wall to provide electrical connections at the outer side of the vessel for connection with the second analytical part comprising the power supply and the controller.

In one embodiment, a plurality, such as an array, of electrochemical sensors is provided within the analytical unit. Each electrochemical sensor may be provided for the detection of a certain analyte, such as a particular protein. Additionally or alternatively each electrochemical sensor may be adapted to detect an analyte at a particular concentration or a particular reactivity (or specificity). Thus, each electrochemical sensor may be optimised for the particular conditions for which it is intended to be used. Each electrochemical sensor is operable simultaneously with or independently of the other electrochemical sensors.

In one embodiment, a plurality of electrochemical sensors is provided to allow for the reuse of the analytical unit.

Working Electrode

The electrochemical sensor includes a working electrode (a first working electrode), which may have an electrically conductive matrix. The electrode may have an electrically conductive carbon- or graphite-containing matrix.

The electrode may comprise an electrically conductive carbon- or graphite-containing matrix holding a first reagent and/or a second reagent. Preferably, the first and second reagents are present. Alternatively, one of the first or the second reagent is present. Where only one of the reagents is provided in the working electrode, the other may be provided in the first chamber of the preparation vessel, to be made available to the electrode surface during the electrochemical analysis.

The electrode may be or comprise a carbon- or graphite-containing matrix. Alternatively the working electrode may be or comprise Pd, Au, Ag, Pt, Fe and mixtures thereof. The working electrode may be or comprise steel.

The working electrode of the present invention is stable. It does not degrade substantially over time or degrade substantially on prolonged exposure to the environment at which it is intended to be used.

The working electrode of the present invention may be stored for at least 14 days, at least 28 days, or at least 6 months without significant loss of electrode activity. Preferably the electrode is stored for this time in a dry state to minimise degradation. It is preferred that the working electrode, and optionally the other components of the sensor, is stored in an atmosphere of an inert gas, such as nitrogen or argon. The preferred carbon paste electrodes for use herein have a shelf life of at least 15 months.

The activity of the working electrode may be gauged by electrochemical analysis of a standard sample solution comprising a protein. A loss of electrode activity corresponds to a fall in the average recorded current recorded at the working electrode in comparison to a recorded current at a control working electrode. Preferably, the fall in average recorded current is about 50 % or less, about 30 % or less, about 10 % or less, or about 5 % or less. The environment against which the stability of the working electrode is tested may include a biological sample as described herein. The working electrode may also be tested against a sample approximating the conditions to which the working electrode is intended to be exposed. The sample may be a simulated intestinal fluid, for example, or a simulated stool sample.

Electrically Conductive Matrix

Generally, the working electrode has an electrically conductive matrix holding, and may hold a first reagent and/or a second reagent. The matrix can be a porous matrix.

The electrode may be an electrically conductive carbon- or graphite-containing matrix.

The electrically conductive matrix is adapted for electrical connection to a voltage supply. Preferably the electrically conductive matrix is electrically connected to a conducting substrate. Preferably the conducting substrate is metal.

The metal may be, for example, steel or platinum, and may be in any shape, although wires (including coils) are most preferred. In use, the conducting substrate may form the electrical connection between the electrically conductive matrix of the working electrode and a voltage supply.

In a preferred embodiment the conductive matrix is, or is obtained or obtainable, from a graphite or carbon paste. In an alternative embodiment the conductive matrix is, or is obtained or obtainable, from a carbon- or graphite-containing ink.

Carbon pastes are well known as such in the art. A carbon paste may be prepared from graphite or carbon particles, and an oil.

The oil may be, amongst others, paraffin, mineral oil or silicone oil. A suitable oil includes a mineral oil available from Sigma-Aldrich (see catalogue number M3516, for example).

Suitable graphite or carbon particles include synthetic graphite powder of average particle size < 20 $\mu$m available from Sigma-Aldrich (see catalogue number 282863, for example).

The paste is prepared by mixing the graphite or carbon particles and oil together, for example using a pestle and mortar or a milling machine, as described herein.

A graphite or carbon paste may also be prepared by melting paraffin, for example a paraffin wax, in the presence of graphite or carbon. Typically, the paraffin is heated to a temperature in the range 40-50°C. Such techniques are described in Petit et al.

Alternatively, a commercially available paste may be used. A suitable carbon paste includes a carbon paste available from Bioanalytical Systems, Inc. (see, for example, catalogue number CF-1010).

The typical composition for a paste is 55-75 wt % graphite or carbon, and 25-45 wt % oil. Preferably the composition is 60-70 wt % graphite or carbon, and 30-40 wt % oil.

The electrically conductive matrix may be a carbon ink.

A carbon or graphite ink typically has a solids content in the range 30-50 wt %, preferably 33-45 wt %. Carbon or graphite ink commonly includes carbon or graphite with a solvent and a binder, such as a vinyl- or epoxy-based polymeric binder.

Carbon and graphite inks are commercially available, and suitable inks for use in the present invention include those inks from Acheson Colloids (see, for example, Electrodag Standard Carbon Ink PF-407A), Dupont Electronic Materials (see, for example, product number BQ242), Gwent Electronic Materials Ltd (see, for example, product number C10903P14) and Ercon.

The electrically conductive matrix may be multilayered. A multilayered electrically conductive matrix allows the first and/or second reagents, and additional additives where present, to be physically separated within the matrix. This has been found to increase the stability of the matrix in comparison to a matrix having the first and/or second reagents, and additional additives where present, within a single layer.

The multilayered conductive matrix minimises the cross reactivity of the components of the matrix. For example, the cross reactivity of the first reagent and the second reagent in the absence of the protein is reduced in the multilayered conductive matrix.

The multilayered electrically conductive matrix may have a layer of carbon paste or carbon ink that does not hold either the first or second reagent. Preferably this layer consists of carbon paste or carbon ink. Where a conducting substrate is present, it is preferred that this layer of carbon paste or carbon ink is attached to the conducting substrate.

Preferably the matrix has a first layer holding the first reagent and a second layer holding the second reagent, where each of these reagents is present.

In a most preferred embodiment, the electrically conductive matrix comprises a first layer of carbon paste or carbon ink, that layer holding one of the first reagent or the second reagent. Adjacent to the first layer is a second layer of carbon paste or carbon ink, that layer holding the other of the first reagent or the second reagent. Adjacent to the second layer, and on the opposite side to the first layer, is a third layer of carbon paste or carbon ink. The third layer may not hold either the first or second reagent, and preferably consists of carbon paste or carbon ink. Optionally, a conducting substrate may be present adjacent to the third layer, and on the opposite side to the second layer.

It is preferred that the first layer is the outer layer that is exposable to the material to be analysed. It is preferred that the first layer holds the second reagent, and the second layer holds the first reagent.

It is preferred that carbon ink is used in a multilayered electrically conductive matrix.

The electrically conductive matrix is porous. The porous network permits, in use, an analyte, such as a protein, to penetrate the matrix where it is made available to catalyse the reaction between a first reagent and a second reagent. Where only one of these reagents is present in the electrically conductive matrix, the pores permit, in use, the other of those reagents to penetrate the matrix where it is made available for reaction with the one reagent catalysed by the protein.

Preferably the pores have an average diameter in the range of at least 1 $\mu$m, at least 5 $\mu$m, at least 8 $\mu$m, at least 10 $\mu$m, at least 15 $\mu$m, or at least 20 $\mu$m. Preferably the pores have an average diameter in the range of at most 8 $\mu$m, at most 20 $\mu$m, at most 25 $\mu$m, or at most 30 $\mu$m.

Preferably the pores have an average diameter in the range of about 8 to about 30 $\mu$m.

The size of pores in an electrically conductive matrix may be determined experimentally by SEM.

First Reagent

In one embodiment, the working electrode holds the first reagent.

The amount of first reagent present in the working electrode is selected to allow the optimal performance of that electrode. The amount of first reagent present is of a quantity sufficient to give rise to a detectable signal upon reaction of that first reagent with the second reagent in the presence of the protein, where the protein is present at physiological or clinical levels. The amount of reagent present is at a concentration that allows sufficient electron transmission through the matrix. The amount of reagent is selected so as to provide optimal sensitivity and response times in use.

Additionally or alternatively, the amount of first reagent present is the amount that gives the optimal structural integrity to the electrically conductive matrix.

The first reagent may be present in the electrically conductive matrix in at least 1 wt %, at least 2 wt %, or at least 5 wt %. The first reagent may be present in the electrically conductive matrix in at least 4 wt %.

The first reagent may be present in the electrically conductive matrix in at most 15 wt %, at most 9 wt %, or at most 5 wt %. The first reagent may be present in the electrically conductive matrix in at most 15 wt %, at most 9 wt %, or at most 6 wt %.

Preferably the first reagent is present in the electrically conductive matrix at 1-15 wt %. Preferably the first reagent is present in the electrically conductive matrix at 2-9 wt %. Preferably the first reagent is present in the electrically conductive matrix at 5 wt %.

Second Reagent

In one embodiment, the working electrode holds the second reagent.

The amount of second reagent present in the working electrode is selected to allow the optimal performance of that electrode. The amount of second reagent present is of a quantity sufficient to give rise to a detectable signal upon reaction of that second reagent with the first reagent in the presence of the protein, where the protein is present at physiological or clinical levels. The amount of reagent present is at a concentration that allows sufficient electron transmission through the matrix. The amount of reagent is selected so as to provide optimal sensitivity and response times in use.

Additionally or alternatively, the amount of second reagent present is the amount that gives the optimal structural integrity to the electrically conductive matrix.

The second reagent may be present in the electrically conductive matrix in at least 2 wt % or at least 7 wt %. The second reagent may be present in the electrically conductive matrix in at least 6 wt %.

The second reagent may be present in the electrically conductive matrix in at most 15 wt %, at most 11 wt % or at most 7 wt %.

The second reagent may be present in the electrically conductive matrix in at most 8 wt %.

Preferably the second reagent is present in the electrically conductive matrix at 2-15 wt %. Preferably the second reagent is present in the electrically conductive matrix at 7-11 wt %. Preferably the second reagent is present in the electrically conductive matrix at 7 wt %.

Counter Electrode

The electrochemical sensor comprises a counter electrode. The counter electrode is connectable to a power source. Preferably, the counter electrode is connected to the power source when the counter electrode is used as a reference electrode.

There are no specific limitations on the type of counter electrode that may be used in the electrochemical sensor of the invention. Electrode materials include carbon paste, steel and platinum. Carbon paste is the most preferred electrode material for use in disposable and one shot sensors and apparatus owing to its relatively low cost and ease of use in a screen printed electrode system.

The counter electrode may be identical to the working electrode save that the counter electrode does not comprises either the first or second reagent.

Reference Electrode

A reference electrode may be included in the electrochemical sensor of the invention.

The reference electrode may be a standard silver / silver chloride electrode. The reference electrode may be a pseudo reference electrode, which is operable as a reference electrode in the presence of a suitable buffer comprising appropriate ions. In one embodiment, the pseudo reference electrode may be a silver-based electrode that is obtained, or is obtainable from, a silver electrode that is treated with about 1% aqueous $FeCl_3$ solution.

In one embodiment, the reference electrode is a silver/silver chloride paste electrode, for example a 60/40 silver/silver chloride paste. Such are preferred for use in disposable and one shot sensors and apparatus owing to its relatively low cost and ease of use in a screen printed electrode system.

Additional Working Electrode

The electrochemical sensor may comprise a second working electrode. The additional working electrode may be the same or different to the first working electrode.

The second working electrode may be provided to permit differential electrochemical measurements to be taken in the use of the electrochemical sensor. The second working electrode may be used in permit duplicate electrochemical measurements to be taken in order to demonstrate accuracy and increase confidence.

The sensor of the invention may be provided in a triplicate or quadruplicate configuration. Thus, the sensor may include two or three further working electrodes.

The addition of a further electrode can improve the overall performance of the electrochemical sensor by allowing a higher specificity for the protein. Thus, the electrode is more capable of discriminating between different types of proteins, for example, between haemoglobin and horseradish peroxidase.

In one embodiment, the second working electrode is provided to measure the uncatalysed reaction between the first and second reagent. In this embodiment, second working electrode may be provided with one of the first or second reagents, and the working electrode may be provided with both the first and second reagents. In the absence of the other reagent, the electrode is capable of providing a base line signal in the absence of the catalysed reaction. During the sample analysis there is insufficient time for the other of the first and second reagent to diffuse from the first working electrode to the second working electrode to provide a catalysed detectable signal.

Electrochemistry

The electrochemical sensor may further comprise a voltage supply (or power supply). The voltage supply is preferably adapted to supply a constant bias between the working electrode and the counter electrode or the reference electrode, where present.

Preferably the voltage supply is adapted to supply a constant bias in the range - 0.05 to + 0.25 V between the working electrode and the reference electrode or counter electrode.

Preferably the voltage supply is adapted to supply a constant bias of about + 0.10 V between the working electrode and the reference electrode or counter electrode.

Preferably the voltage supply is adapted to supply a constant bias of about + 0.03 V between the working electrode and the reference electrode or counter electrode.

The electrochemical sensor may further comprise a detector for monitoring current.
The electrochemical sensor may further comprise a controller for controlling the voltage supply and timing of that supply.

In one embodiment, the electrodes (working, counter and reference) are provided in the preparation vessel as a first part of the analytical unit. The analytical unit may comprise a second part of the analytical unit. That second part may comprise the power supply, optionally together with a detector and a controller. The first part and the second part are electrically connectable.

Alternatively the voltage supply and/or the controller may be provided externally. In one embodiment the voltage supply and/or the controller may be a component of an apparatus, such as those apparatus described herein, and in particular as a component of a first module of the apparatus as described herein.

This is particularly preferred where the apparatus, or the first module of the apparatus, comprises an array of electrochemical sensors of the invention. One or more, or each, of the electrochemical sensors of the array may be supplied from a common voltage supply and/or may be controlled by a common controller.

Data Transfer

In one embodiment, the analytical unit is provided with means for transmitting recorded analytical data to a computer. The recorded data may be transmitted using technologies such as wireless communication (e.g. Bluetooth). Alternatively, the analytical unit may be docked with a computer using wired technology (e.g. USB ports). In another embodiment, the analytical data may be stored to a removable memory device of the analytical unit. The memory device may be removed from the analytical unit and docked with the computer for data transfer to the computer.

In one embodiment, the personal test device is provided with a port for connection for electrical connection between the analytical unit and a computer.

The data held by the analytical unit may be transferred to the personal computer of the user to build up a record of test results over multiple test samples. The data may also be uploaded to or transferred to a computer of a healthcare professional, such as a physician, for establishing a medical record for the subject from whom the sample is taken. The healthcare professional may make a diagnosis on the basis of one or more analysed samples, and may communicate a diagnosis and/or a treatment to the user or the subject, for example via the device itself.

The method of the invention may therefore further comprise the step of transmitting analytical data from the analytical unit to a computer. The transmission may be wireless transmission.

*Kit*

Described herein is a kit comprising one or more of the sampler, preparation vessel and the analytical unit of the invention.

In one embodiment, the kit may comprise a set of instructions describing a method for the use of the one or more of the sampler, preparation vessel and the analytical unit. The method may be a method of the invention. The instruction may be provided as a pamphlet, or may be provided on a memory device, such as a CD or a memory stick.

Additionally or alternatively, the kit may be provided with directions (a hyperlink) to instructions that are provided on the internet. Where the analytical unit is provided with a display, the instructions for use may be provided on the display.

The kit may include a record sheet for the user to note down the result or results obtained from the analysis. This record sheet may be in the form of a pamphlet or may be an editable record held on a computer. The record sheet may be provided on a memory device, or may be accessible via the internet.

The kit may comprise a glove, preferably a pair of gloves, for use by the user during the sample analysis. The glove or gloves may be surgical style gloves. The gloves may be provided where the sample for analysis is potentially hazardous or is unpleasant to the user.

The kit may comprise a plurality of reaction vessels. Multiple preparations vessels may be provided to allow a user to perform repeat or multiple sample measurements. In one embodiment, the number of samplers in the kit, and the number of gloves, where present, is equal to the number of preparations vessels. Thus, a sampler and a glove (or pair of gloves) is used only once together with a single preparation vessel. For subsequent measurements, a further set of preparation vessel, sampler and glove may be used. The use of further samplers and gloves ensures there is no contamination of earlier biological samples into the subsequent samples.

In one embodiment, parts of the kit are provided in sealed packaging to minimise exposure to water and/or air. Thus, in one embodiment, the kits or one or more of the sampler, analytical unit and preparation device are provided in a sealed package. The package may hold the components in an anhydrous or anaerobic atmosphere. The package may provide a desiccant material within. The atmosphere may be an inert atmosphere, such as a nitrogen or argon atmosphere. Alternatively, the package may be vacuum sealed.

However, the personal test device of the present invention may be reliably stored long term without the need for an inert atmosphere, or with the need for vacuum. In the preferred embodiments, of the invention, the personal test device is stored together with a desiccant to absorb water.

The packaging is one capable of maintaining an anhydrous or anaerobic atmosphere within. Foil packaging, such as aluminium foil packaging, is suitable for use.

In a further embodiment, the kit is, or is provided with, a sample collector for collecting a faecal deposit from the test subject. The sample collector may be adapted for placement across or within a toilet pan, or any other related device such as a commode. The sample collector may be a sling. The sample collector is provided with means for securing the sample collector to the toilet, including ties, adhesive surfaces and the like. The sample collector and the securing means, where present, are sufficient to support the weight of a deposited faecal sample. In many cases, it is sufficient to use a paper sample collector, which is suitable provided with securing means. Such sample collectors are familiar to those of skill in the art.

The sampler may be used to extract a faecal sample directly from the sample collector. The sample collector may be subsequently flushed to waste. Thus, the sample collector is preferably of a material that will decompose or disintegrate under standard waste treatment processes. As noted, above paper may be use

### Other Preferences

Each and every compatible combination of the embodiments described above is explicitly disclosed herein, as if each and every combination was individually and explicitly recited.

Various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures described above.

### Exemplary Test Devices of the Invention

The present invention provides a personal test device for analysing a sample, particularly a biological sample.

A first part of an analytical unit for use in a personal test device according to one embodiment of the invention is shown in Figure 1(f), which is prepared from the substrate shown in Figure 1(a). The analytical unit is suitable for use with the preparation vessel and sampler of Figure 2, as described below.

The first part of the analytical unit of Figure 1(f) has a series of four electrodes on the top surface of a non-conducting substrate, such as a sheet of non-conducting polymeric material. The electrodes are electrically connectable to a second part of the analytical unit (not shown), which comprises a power supply, a controller and other components associated with an electrochemical sensor. The electrodes are a first working electrode, a second working electrode (which is optional), a counter electrode and a reference electrode. Each electrode is electrically connected to a conducting track 3, which is also provided on the top surface of the substrate. The conducting tracks are suitable for making contacts with corresponding electrical connectors provided on the second part of the analytical unit. The conducting tracks 2 are covered in part with a non-conducting protective layer 3, which may also cover other top surfaces of the substrate, except for the electrodes which remain available for electrochemical reaction. A region 4 of the conducting tracks 3 is exposed and available for forming electrical connection with the second part of the analytical unit.

The electrodes are covered with an absorbent pad 12 that is a permeable sponge. In use, the sponge is suitable for absorbing material, including electrolyte and sample, and holds the material close to the electrode surfaces, where it is available for electrochemical reaction. Advantageously, the sponge holds the material to the electrode surfaces when the part is inverted. The sponge also acts as a filter, preventing solids from coming into contact with the electrode surfaces, and therefore prevents those surfaces from becoming blocked in use.

The part of the analytical unit of Figure 1 (f) may have a first working electrode comprising a first reagent and a second reagent, such as TMB and perborate. The reaction of these example first and second reagents is catalysed by haemoglobin, and the electrode array may be used to detect the presence of haemoglobin, and therefore blood, in a sample, such as a faecal sample.

The first part of the analytical unit is prepared from the patterned substrate shown in Figure 1(a). The patterned substrate includes a substrate 1 having four conducting tracks 2 provided on it. The conducting tracks 2 provide the electrical connections on the substrate between the electrodes and the second part of the analytical unit (which includes the power supply and the controller, for example). The electrical tracks 2 are films of conducting carbon that are deposited, for example printed, as an ink on the substrate a surface. As shown in Figure 1(a), four separate tracks are provided for each of the four electrodes that are to be provided on the substrate 2.

After the tracks are laid, a non-conducting protective layer 3 is provided across the top surface of the patterned substrate of Figure 1(a). The layer 3 is provided with suitable through channels that allow regions of each track to remain exposed. These exposed regions are the locations for forming the electrodes and the regions for connection with the second part of the analytical unit. As shown in Figure 1(b), exposed region 4 is available for forming electrical connection with the second part of the analytical unit. Exposed regions 5, 6, 7 and 8 are the locations for forming the counter electrode, second working electrode, first working electrode and reference electrode respectively.

The subsequent steps in the method are the preparation of the working and reference electrodes. Thus, in a first step, a conductive silver/silver chloride paste may be printed onto the exposed region 8 to give the reference electrode 9. Thus, the reference electrode 9 is in electrical connection with a conducting track 2. The intermediate unit is shown in Figure 1(c).

In a subsequent step, a carbon paste mixture may be printed onto the exposed region 6 to give the second working electrode 10, and a carbon paste mixture containing first and second reagents, such as TMB and perborate, may be printed onto the exposed region 7 to give the first working electrode 11. Thus, the first and second working electrodes 10 and 11 are in electrical connection with a conducting track 2. The intermediate unit is shown in Figure 1(d).

The counter electrode may be formed by providing a suitable conducting material on the exposed region 5. However, in many cases, the exposed conducting track at region 5 may itself be suitable for use as a counter electrode. Therefore, no further modification is required.

The electrodes are them provided with an absorbent pad 12, which covers all the electrode surfaces. As noted above, the purpose of the absorbent pad is to draw fluid to the electrodes and to act as a filter preventing solids from blocking the electrode surfaces. The intermediate unit is shown in Figure 1(e).

As a final step, a non-conducting top sheet 13 is provided over the absorbent pad. The top sheet covers the entire absorbent pad, except for an opening that defines a sample entry hole to the absorbent pad, a part of which is exposed at the opening.

In use, the part of the analytical unit of Figure 1(f) is connected to the second part of the analytical unit, which provides the power supply and the controller. The connection is formed to exposed regions of the track 4. In use, these regions may be shielded from the material to be analysed.

Material to be analysed is provided to the electrode surfaces, and electrochemical analysis is performed, using the electrodes as is well understood by a person skilled in the art. The material to be analysed is supplied from the chamber of the preparation vessel. For electrochemical analysis, the material includes an electrolyte. The material is permitted to come into contact with the absorbent pad 12 which is exposed at an entry hole in the non-conducting top sheet 13. Fluids in the material are drawn through the absorbent pad and come into contact with the electrodes. Appropriate electrochemical analysis may then be performed. In some embodiment, it is not necessary to have an absorbent pad, and material may be placed directly on the electrode surfaces from the preparation vessel.

The first part of the analytical unit of Figure 1 (f) is designed to be a one use, disposable part of a personal test device. Once the part has been used, it may be discarded. The analysis of further samples may be undertaken using further analytical units of formula 1(f). Advantageously, the second part of the analytical unit, may be simply disconnected from a used first part of the analytical unit and may be attached to a unused first part of the analytical unit, for further analysis.

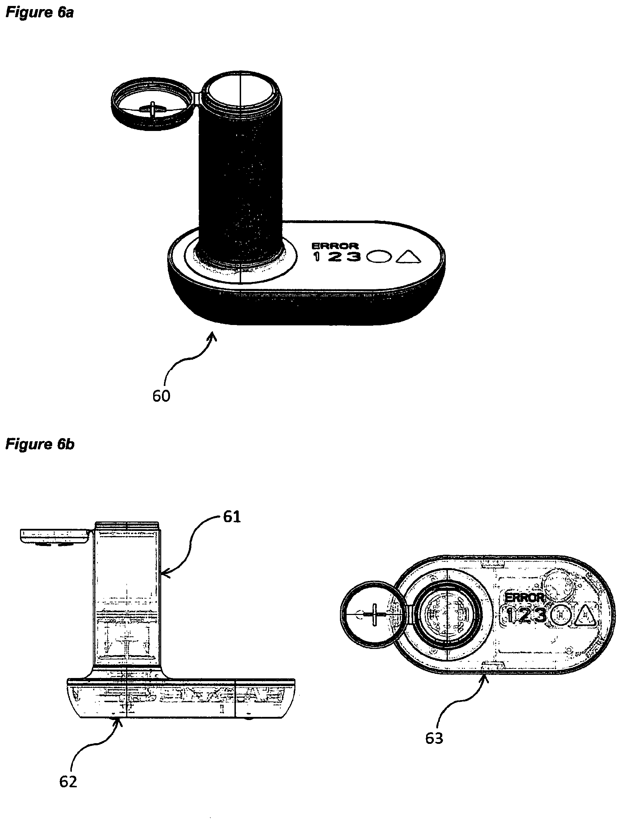

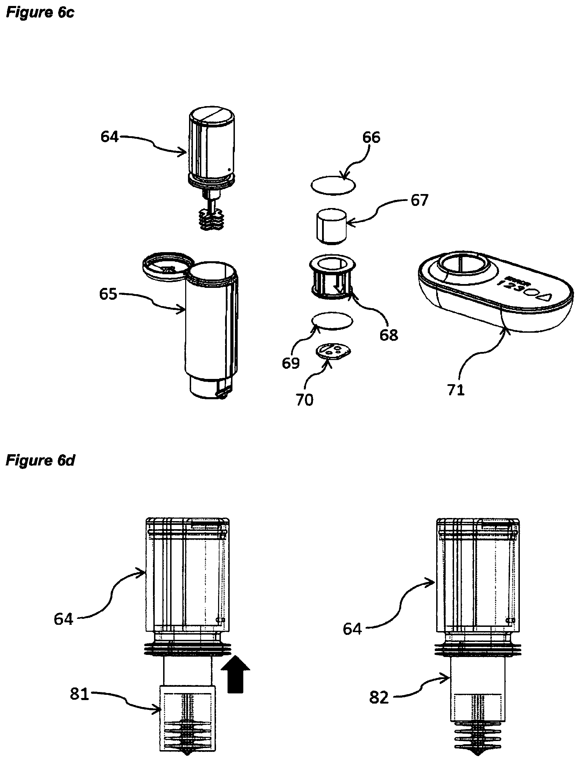

The first part of the analytical unit of Figure 1 (f) may be adapted for use in a personal test device where the sampler, preparation vessel and analytical unit are held together as a single unit. Such a personal test device is shown in Figure 6, where the first part of the analytical unit 70 is similar to that of Figure 1 (f).

Figure 2 shows a sampler 20 and a preparation vessel 22 according to some embodiments of the invention. Figure 2(a) shows the sampler 20 and a cross section of the preparation vessel 22. Figure 2(b) shows the sampler 20 prior to its engagement with and insertion into the preparation vessel 22. Figure 2(c) shows the sampler 20 inserted into the preparation vessel 22.

The sampler 20 comprises a shaft 22 having a piercing tip 23 at one end. The shaft 22 is substantially cylindrical. The section of the shaft 22 distal to the piercing tip 23 may be used as a handle by the user. The shaft is provided with a series of bristles 24, which extend radially from the longitudinal axis of the shaft 22. The bristles 24 are spaced apart from the piercing tip on the shaft 22. The shaft 22 is also provided with a seal 25. The bristles 24 are located on the shaft 22 between the piercing tip 23 and the seal 25. The distance between the piercing tip 23 and the seal 25 is equal to or greater than the distance between the first and second seals 27,29 of the preparation vessel 21, as described below.

The bristles 24 are provided around the circumference of the shaft 22. The bristles 24 also extend along the shaft 22. The distance the bristles 24 extend along the shaft 22 is less than the length of the chamber 28 in the preparation vessel 21.

The bristles 24 radially extend from the shaft 22 to a distance that is greater than the radial distance extended by the piercing tip 23. In another embodiment, the bristles 24 and the piercing tip may extend in the radial direction by the same distance. The bristles 24 are flexible, which allows the bristles 24 to be passed through a piercing in the seal of the preparation vessel that has a smaller cross-section distance than the cross-section distance (diameter) of the sampler 22.

The seal 24 may be an O-ring seal. The seal 24 radially extends from the shaft 22 to a distance that is greater than the radial distance extended by the piercing tip 23. The distance from the piercing tip 23 to the seal 25 may be equal or greater than the distance between the first and second seals of the preparation vessels.

The sampler shown in Figure 2 is suitable for holding a solid sample, such as a faecal sample.

For use together with the sampler 22 is a preparation vessel 21, which is shown in a cross-section perspective in Figure 2(a). The preparation vessel 21 is generally cylindrical having a first opening 26 at one end face and a second opening 30 at the other end face. The first opening 26 is provided with a seal 27 and the second opening 30 is provided with a second seal 29. The preparation vessel has a chamber 28. The chamber 28 holds one or more reagents, and where the preparation vessel is for use together with an analytical unit such as shown in Figure 1(f), the chamber holds an electrolyte. The first and second openings 26, 30 are connected with the chamber 28. The first seal 27 and the second seal 29 prevent material from entering or leaving the chamber 28 via the first and second openings. When the first and second seals 27, 29 are pierced, for example by the sampler 20, the first and second openings 26, 30 provide fluid communication between the chamber 28 and the outside of the vessel.

The chamber 28 is adapted to receive the protrusions, such as bristles 24, of the sampler 20. The dimensions of the chamber 28 are therefore such that allow the region of the sampler 20 having the protrusions to be accommodated within. Typically, the chamber 28 is of sufficient size to allow the protrusions to extend to its greatest radial distance from the shaft, as explained below.

The first and second openings 26, 30 may have a substantially circular cross-section. Similarly, the sampler 20 may also have a circular cross-section. The first and second openings 26,30 are sufficiently large to allow the piercing tip 23 and the shaft 22 to pass through.

The first opening 26 may be tapered from the outside wall of the preparation vessel 21 to the chamber walls. In use, the tapered opening guides the piercing tip 23 of the sampler 22 to the first seal 27. The second opening 30 may have a constant cross section along its length.

Figure 2(a) shows the preparation vessel 21 held upright with the first opening 26 above the second opening 30. Figure 2(b) shows a perspective view of the sampler 20 poised above the preparation vessel 21.

The sampler 20 is used to hold a sample for analysis. For example, the sampler 20 may be used to hold a faecal sample for delivery into the chamber 28. Here, the bristles of the sampler 20 are brought into contact with a faecal sample from a subject. The faecal material is thereby transferred to the protrusions, such as bristles 24, and it is held on the protrusions, in-between protrusions, and in-between protrusions and the shaft 22. Faecal material may also be held on the piercing tip 23 and the shaft 22.

After the sampler 20 is suitably loaded with sample, the sample is delivered into the chamber 28. Figure 2(b) shows the sampler 20 in preparation for its insertion into the preparation vessel 21. In use, the piercing tip 23 is placed above the first opening 26 of the preparation vessel 21. The piercing tip 23 is pushed into the first opening until it contacts the first seal 27. The tapered first opening guides the movement of the piercing tip 23 to the first seal 27. The piercing tip 23 is forced against the first seal 27 until the first seal 27 is pierced (broken). The piercing tip 23, followed by the shaft 22, is then pushed into the chamber 28. As the user continues to push the sampler into the preparation vessel 21, the bristles 24 pass through the first opening 26 and into the chamber 28 where the bristles 24, together with the piercing tip 23 and regions of the shaft 22, come into contact with the reagents, such as electrolyte, held within the chamber 28.

As the sampler is pushed through the first opening 26, the bristles 24 of the sampler 22 contact the side walls of the first opening 26. The bristles 24 are flexible and are swept back against the shaft 22. With the bristles 24 swept back the sampler 22 is further pushed through the first opening, with the bristles 24 passing through the piercing in the first seal 27. As the bristles 24 pass through the piercing and into the chamber 28, the bristles 24 flex back to their original extended position (i.e. to their greatest radial distance from the shaft). The flexing action of the bristles 24 as they enter the chamber 28 is beneficial. The flexing action causes material held on and between the bristles 24 to be flicked from the bristles into the reagents, such as an electrolyte. In this way sample is efficiently disturbed into the reagents. The flexing action of the bristles 24 also provides a mixing action for the reagents, together with sample, in the chamber 28.

The reagents in the chamber 28 contact the sample on the sampler 22. The sample may be released from the sampler 22 by the flexing action of the bristles 24. The user may also assist the distribution of sample in the chamber 28 by gently shaking the preparation vessel 21 and/or movement of the sampler 22 in the chamber 28.

The one or more reagents in the chamber prepare the sample for analysis by the analytical unit. For example, where the sample is a faecal sample, a ell lysing reagent may be provided to lyse any blood cells present. In this way, haemoglobin is released from the cells, and may be detected by the analytical unit. As described above, the analytical unit of Figure 1(f) may be used be to electrochemically detect haemoglobin on the basis of its catalytic activity. The mixture of the reagents and the sample is referred to as material. It is noted that the reagents may bring about a chemical and/or physical change in the sample, and therefore the material may be more than simply a mixture of the reagents with the sample.

The user may continue to push the sampler 22 into the preparation vessel 21. The piercing tip 23 will then come into contact with the second seal 29, which is located within the second opening 30. The piercing tip 23 is forced against the second seal 29 until the second seal 29 is pierced (broken). The piercing tip 23 may then pass further along the second opening. With the first seal 27 and the second seal 29 pierced, material from the chamber 28 may pass through either the first or second opening and out of the preparation vessel.21. Where, the first opening 26 is held above the second opening 30, as shown in Figure 2, the material will pass out of the second opening 30.

The movement of the sampler 20 through the preparation vessel 21 is limited by the presence of a seal 25 on the shaft 22 of the sampler. As the sampler 22 passes through the vessel, the seal 25 is brought into contact with the preparation vessel 21. The size of the seal 25 is such that it cannot pass through the first opening into the chamber. Thus, once the seal 25 contacts the preparation vessel 21, further movement of the sampler 20 through the preparation vessel 21 is prevented. The seal 25, together with the shaft 22, is adapted to entirely block, or seal, the first opening 26. Thus, in this arrangement, which is shown in Figure 2(c), material from the chamber 28 is prevented from exiting the preparation vessel 21 via the first opening 26, and instead passes out of the chamber 28 via the second opening 30.

Thus, after the second seal 29 is pierced, material may flow from the chamber 28 through the second opening 30 and out of the vessel 21. In use, the vessel 21 is held with the second opening 30 above the analytical part. Thus, material drops from the vessel onto an appropriate area of the analytical part, for example the absorbent pad 12 of the analytical part of Figure 1(f).

The sampler and the preparation vessel are designed to be one use, disposable parts of a personal test device. Once the sampler and the preparation vessel have been used, they may be discarded. The analysis of further samples may be undertaken using further samplers and further preparation vessels.

In principal, used samplers and used preparation vessels may be collected, cleaned to a suitable level for medical use, and prepared for re-use. For the domestic or medical setting, such steps may be particularly onerous, and it is preferred that the sampler and the preparation vessel are disposable.

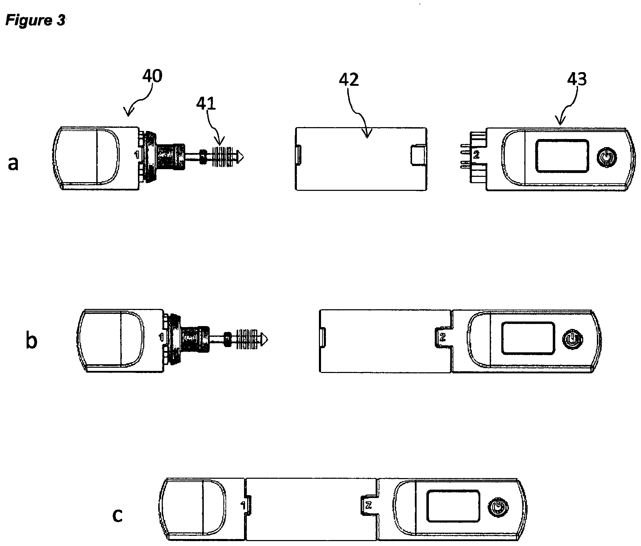

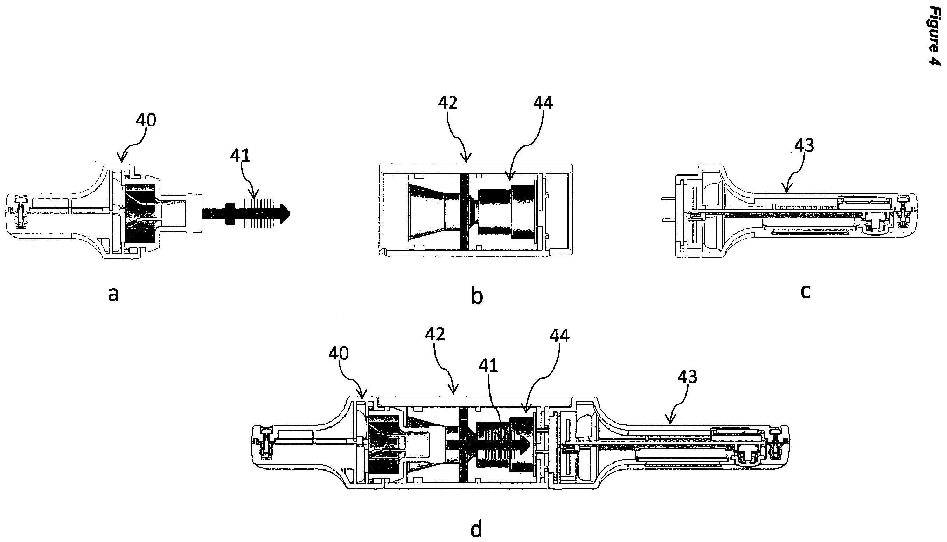

Figures 3 and 4 show a personal test device according to an embodiment of the present invention. Figure 3 is a perspective view, and Figure 4 is a cross-section view of the test device. The test device comprises a separate sampler 40, a preparation vessel and first analytical part 42, and second analytical part 43, as shown in Figure 3(a) and 4(a). The sampler 40, preparation vessel and first analytical part 42, and second analytical part 43 are individual units that may be connected together in a unified device, as shown in Figure 3(c) and Figure 4(b). Typically, in use, the preparation vessel and first analytical part 42, and second analytical part 43 are brought together, as shown in Figure 3(b), and then the sampler 40 is brought into contact with the preparation vessel, thereby to form the unified device of Figure 3(c).

The sampler 40 shares many of the features of the sampler 20 shown in Figure 2. Thus, the sampler 40 comprises a shaft having a piercing tip at one end of the shaft and protrusions, such as bristles 41, which are spaced apart from the piercing tip along the shaft. The protrusions may be provided around the circumference of the shaft. The protrusions also extend along the shaft. The distance the protrusions 24 extend along the shaft 22 is less than the length of the chamber 44 in the preparation vessel 42. The protrusions may radially extend from the shaft to a distance that is greater than the radial distance extended by the piercing tip.

The shaft of the sampler 40 is provided with a handle which is located at the end of the shaft distal to the piercing tip. The handle provides the user with a more secure grip for the sampler. The shaft is provided with a first seal. The bristles 41 are located on the shaft between the piercing tip and the first seal. The sampler is also provided with a second seal at the join of the shaft with the handle.

Also provided is a preparation vessel and first analytical part 42.comprising a first chamber connected to a second chamber 44 *via* a second opening. The first chamber 28 holds one or more reagents in the form of an electrolyte. The second seal prevents material from the first chamber from entering the second chamber 44. When the second seal is pierced, for example by the piercing tip of sampler 40, the second opening provides fluid communication between the first chamber and the second chamber 44.

The second chamber 44 is provided with a first analytical part for analysing material in the second chamber. A wall of the second chamber is provided with electrodes, such as working, counter and reference electrodes, for the electrochemical analysis of material in the second chamber 44. The electrode surfaces are exposed to contents of the second chamber. The electrodes are also electrically connected to the outer surface of the vessel 42. In preferred embodiments of the present invention, the electrodes are adapted for detection of haemoglobin in a sample. The haemoglobin is detectable from its ability to catalyse the reaction of a first reagent with a second reagent. The first and second reagents may be provided within the working electrode. When the material from the first chamber enters the second chamber, the surface of the working electrode is wetted, allowing the first and second reagents to be made available for reaction with haemoglobin in the mixture, is such is present.

The preparation vessel and first analytical part 42 is generally cylindrical and has a first opening at one end face. The first opening is provided with a seal. The first opening is connected with the first chamber. The first seal prevents material from leaving the vessel 42. When the first seal is pierced, for example by the piercing tip of sampler 40, the first opening provides fluid communication between the chamber and the outside of the vessel. The first opening and the first chamber have larger cross-sections than the second opening. The first seal of the sampler has a smaller cross-section than the first opening and the first chamber. The first seal has a larger cross-section than the second opening. The second seal has a larger cross-section that the first opening.

Also provided is a second part 43 of an analytical unit. This part comprises a power unit, a controller and a visual display suitable for use together with the electrodes of the part 42. The second part 43 is connectable with the vessel and first analytical part 42. The electrodes are electrically connectable to the power unit and the controller. Electrical contacts are provided on an outer surface of the second part 43 of the analytical unit. These contacts are suitable for making an electrical connection with the electrical connections provided on the outer surfaces of the vessel 42.

In use, the second part 43 is connected to the vessel 42. The power unit and the controller are electrically connected to the electrodes. The second part 43 is releasably held with the vessel 42 by any suitable means, including clasps, magnets, or interlocking parts.

The sampler is suitably loaded with a sample, such as a faecal sample, and the sample is added to the first chamber in a manner similar to that described above for the sampler 20 and preparation vessel 21. The piercing tip of the sampler 40 is pushed against the first seal of the vessel 42 until the seal breaks. The sampler is then pushed through the first opening until the bristles 41 are provided in the first chamber. The sample is thereby exposed to the reagents, such as electrolyte, in the chamber.

The sampler is pushed further into the vessel until the piercing tip contacts the second seal. The sampler is pushed through the second seal until the seal breaks. With the second seal broken, material from the first chamber is permitted to flow through the second opening into the second chamber. Material in the second chamber contacts the electrodes and the electrochemical properties of the material may be determined under the control of the controller of the second part of the analytical unit.

After the second seal is pierced, the sampler may be pushed further into the vessel 42 until the first seal of the sampler contacts the vessel, for example the walls of the first chamber at the second opening. The first seal prevents the sampler from passing further into the vessel. The first seal also seals the second opening, preventing material within the second chamber passing back into the first chamber.

As the first seal of the sampler contacts the vessel, the second seal of the sampler also contacts the vessel at the first opening. The second seal also prevents the sampler from passing further into the vessel. The second seal also seals the first opening, preventing material within the first chamber from exiting the vessel via the first opening. The sampler may be releasably held with the vessel 42 by any suitable means, including clasps, magnets, or interlocking parts.

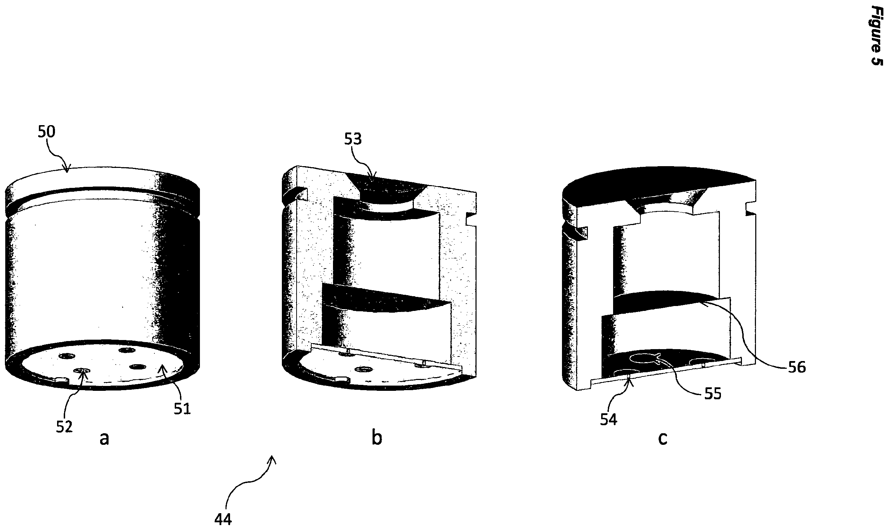

Figure 5 shows a perspective view of an alternative preparation vessel and first analytical part 50 for use in the personal test device of Figures 3 and 4. Figure 5(a) is a perspective view of the underside of the preparation vessel and first analytical part 50. The vessel 50 is generally cylindrical in shape, having a first opening at one end face and a first analytical part provided within the wall of the other end face. The first opening is sealed by a seal 53. The first analytical part comprises a series of four electrodes 52: a first working electrode, a second electrode, a reference electrode and a counter electrode, with each electrode having an electrical contact at the outer surface of the other end face. The electrical contact is suitable for forming an electrical connection to a second part of an analytical unit, which second part comprises a power supply and a controller for the electrodes. The electrode surfaces are exposed to a second chamber in the preparation vessel, as described further below.

Figure 5(b) shows a cross-section perspective view of the underside and side of the preparation vessel and first analytical part 50 of Figure 5(a). The vessel comprises a first chamber, which is connected to a second chamber via a second opening 56. The second opening 56 is sealed. The first chamber is provided with a first opening 53 which is located at one end face of the vessel. The first opening 53 is sealed. Within the first chamber there is provided one or more reagents, which may include an electrolyte.

Figure 5(c) shows a cross-section perspective view of the upper side and side of the preparation vessel and first analytical part 50 of Figure 5(a). As before, the seals at the first and second openings 53, 56 are visible. Within the walls of the second chamber are electrodes 54 and 55, whose surfaces are exposed to the contents of the second chamber. The electrodes 54 and 55 are in electrical contact with the outer side of the vessel at the second end face. The electrodes and the electrical contacts are components of the first part of the analytical unit.

In use, the first seal at the first opening 53 is pierced by a sampler holding a sample, such as the sampler 40 of Figure 4(a), and a part of the sampler is permitted to enter the first chamber. Reagents within the first chamber interact with sample to provide material that is suitable for analysis. The sampler is pushed further into the vessel and is allowed to pierce the second seal of the second opening 56. Material in the first chamber is then able to pass through the piercing into the second chamber, where it comes into contact with the electrodes of the first part of the analytical unit. Prior to the introduction of the sampler to the vessel, the first part of the analytical unit electrically connected to a second part of the analytical unit, such as the second part of the analytical unit 43 of Figure 4(c), which includes a power unit and controller. Thus, once material in the second chamber contacts the electrodes, appropriate electrochemical analysis may be undertaken.

Figure 6 shows a further personal test device 60 according to an embodiment of the invention. Figure 6(a) is a perspective view, Figure 6(b) is a side view and Figure 6(c) is a plan view of the personal test device 60. The device 60 comprises a cylindrical housing 61 that is in connection with a second part of an analytical unit 62. The cylindrical housing is open at the upper end face, though this opening may be closed by placing a cap over the end face. A cap is shown attached to the cylindrical housing 61. The cylindrical housing is provided to hold a preparation vessel and a first part of the analytical unit, as described in further detail below. The cylindrical housing 61 is connected to in connection with a second part of an analytical unit 62 at the lower end face of the cylindrical housing 61. The lower end face is open and exposes the contents of the housing to the second part of the analytical unit. As explained in detail below, a first part of the analytical unit which is present within the cylindrical housing may therefore contact, such as electrically contact, the second part of the analytical unit 62.

The use of the personal test device of Figure 6 is described in the worked examples.

The second part of the analytical unit 62 comprises a housing 63 holding suitable control and power components for use together with the first part of the analytical unit. As described below, the personal test device 60 is suitable for use in the electrochemical analysis of test samples, therefore the second part of the analytical unit 62 may comprise the power supply and controllers for use together with electrodes that are provided in the first part of the test unit. The housing 63 is provided with a display on its upper surface. The display provides the user with a visual indicator of the test result. This may be displayed in the form of coloured lighting, for example a red circular light be indicative of a bad test result, whilst a green triangular light may be indicative of a good test result. Similarly, the display may be provided with indicators elements for indicating an error in the analysis, or indicator elements to guide the user through a timing sequence for the use of the personal test device.

The personal test device 60 may take the form of a device for the electrochemical analysis of a sample. Such a device is shown in Figure 6(c) as a perspective view of a disassembled personal test device.

The personal test device comprises a sampler 64, a preparation vessel 66-69, a cylindrical housing 65, a first part of an analytical unit 70, and a second part of an analytical unit 71. The first and second parts 70,71 of the analytical unit are electrically connectable. At least part of the sampler is insertable into the preparation vessel 66-69. The sampler 64, preparation vessel 66-69 and the first part of the analytical unit 70 are insertable into the cylindrical housing 65. The cylindrical housing 65 is connectable to the second part of a test device 71.

The first part of the analytical unit 70 is a disk having electrodes on its upper surface. These electrodes are in electrical communication with contacts on the lower surface of the disk. These contacts are for connection with corresponding contacts on the second part of the analytical unit 71. The second part of the analytical unit 71 provides power to the electrodes, *via* the contacts, when the units 70 and 71 are connected. The basic construction of the disk is not dissimilar to the electrode set up shown in Figure 1. Thus, the disk is provided with a first working electrode, a second working electrode a counter electrode (which may simply be a carbon electrode) and a reference electrode (typically silver chloride based).

The sampler 64 is shown in more detail in Figure 6(d), which includes side views of the sampler. The sampler 64 has a shaft with a piercing tip at one end. The other end of the shaft is connected to a handle 64. The shaft is provided with protrusions (which may be broadly referred to as bristles) for holding a solid or semi-solid sample. The protrusions may be inflexible. The protrusions are provided on a part of the shaft that extends from the piercing tip. The handle is provided with a sheath 81,82 which is extendable from the handle to at least partly cover the shaft. In Figure 5(d), the right-hand side image is of a sample where the sheath 82 is retracted. The shaft, together with protrusions and the piercing tip, are exposed and are suitable for use in obtaining a sample. With the shaft exposed, the sampler may be used to pierce a preparation vessel, such as 66-69 described below, and to deliver a sample held on and/or amongst the protrusions to a chamber of the preparation vessel.

Figure 6(c) shows the components of a preparation vessel 66-69 for use together with the sampler 64. The preparation vessel comprises a substantially cylindrical vessel 67 having open end faces. The end faces are sealable with seals 66 and 69. The upper end face may be referred to as the first opening having a first seal 66, and the lower end face may be referred as the second opening having a second seal 70. One or more reagents, including an electrolyte, may be contained within the preparation vessel.

In use, a user takes the sampler 64 and inserts the piercing tip, shaft and/or protrusions into a faecal sample. The amount, such as mass, of sample taken up onto the sampler may be measured (e.g. by mass difference measurements before and after sampling). The part of the sampler including the piercing tip is inserted into the cylindrical housing 65, which is attached to the second analytical part. The housing holds the preparation vessel 66-69 and the first part of the analytical unit, which is in electrical communication with the second analytical part. The housing, preparation vessel and first and second parts of the analytical unit are assembled prior to use.

The piercing tip of the sampler 64 pierces the first seal 66 of the preparation vessel and part of the sampler is permitted to pass into the preparation vessel, allowing faecal matter contained on the sampler to contact the reagent contained within the preparation vessel. Further insertion of the sampler 64 allows the piercing tip to pierce the second seal 69. Material within the preparation vessel is permitted to leave the preparation vessel, *via* the opening in the seal, and pass onto the electrodes of the first part of the analytical unit 70. An electrochemical analysis of the mixture, controlled by the second part of the analytical unit 71, is undertaken, and the results of the analysis are stored within the memory of the second part of the analytical unit 71 for later transfer to a computer.

After use, the sampler 64, the preparation vessel 66-69 and the first part of the analytical unit 70 may be discarded, optionally together with the housing 65. The second part of the analytical unit 71 may be retained for further use.

The personal test device of Figure 6 may be stored as shown in Figure 6a, with the sampler 64, preparation vessel 66-69 and the first part of the analytical unit 70 within the housing 65. The housing may be attached to the second part of the analytical unit. Here, it is necessary for the piercing tip of the sampler to be retracted in order to prevent it piercing the first or second seals of the preparation vessel (as shown in Figure 6d). Alternatively, the parts of the personal test device may be stored in a partly assembled or disassembled state, for example within appropriate packaging, such as sealed packing for long term storage.

*Examples*

The present inventors have prepared personal test devices including the first part of the analytical unit of Figure 1(f). The first part may be referred to as a screen printed electrode, in the light of its method of preparation.

Also described below is an electrolyte for use as the one or more reagents for the first chamber. The electrolyte is suitable for preparing a stool sample for analysis by an electrochemical sensor, such as a sensor comprising the first part of the analytical unit of Figure 1(f).

*Electrode Material Preparation*

Working electrodes were prepared from carbon paste and additional reagents.

Blend Formulation and Preparation

A Mixer Mill 200 (manufacturer: Retsch) was used for blend preparations (grinding or mixing). The accessories used together with the MM200 included:

| Grinding/mixing jars | Material | Volume |
|---|---|---|
| Jar (1) | Agate | 5 mL |
| Jar (2) | Agate | 5 mL |
| Jar (3) | Agate | 5 mL |

(continued)

| Grinding/mixing balls | Material | Ball diameter |
|---|---|---|
| Ball (1) | Agate | 9 mm |
| Ball (2) | Agate | 9 mm |
| Ball (3) | Agate | 9 mm |

The jars are push-fit and are for use in performing radial oscillations in a horizontal position. The inertia of the grinding balls causes them to impact with high energy on a material at the rounded ends of the grinding jars, resulting in the pulverization of the material. In addition, the movement of the grinding jars combined with the movement of the balls can result in the intensive mixing of the mixture. More details of the blending operation is provided at the Retsch website (http://www.retsch.com/).

The mixing/grinding balls are provided by the supplier with polished surface (for aesthetic reasons). However, the surfaces of the mixing/grinding jars are not polished mainly due to the difficulty in the polishing process.

Details of the chemicals used for blend formulation and preparation are listed in the table below:

| Chemicals | Supplier | Product number |
|---|---|---|
| 3,3,'5,'5 Tetramethylbenzidine(TMB), $\geq$99% | Sigma | 860336 |
| Sodium Perborate Monohydrate (PER), 20-100 mesh | Sigma | 372862 |
| Carbon Paste with an Oil Base (CP) | BASi | CF-1010 |
| Carbon Paste with an Oil Base (CP) | ModeDX | - |

Jar (1) was used to grind TMB. Jar (2) was used to grind PER. Jar (3) was used to mix and blend carbon paste (CP)/TMB/PER. Jar (3) was also used for mixing CP/TMB and making ModeDX carbon paste.

Carbon Paste Preparation

The ModeDX formulated carbon paste was prepared from a) Graphite powder, Sigma, Cat No: 282863, <20$\mu$m, synthetic; and b) Paraffin oil, Sigma, cat no: 76235-500 mL, Fluka, for IR Spectroscopy;

The method of preparation included the following steps:

(a) 0.35 g graphite powder was poured into a mixing jar and then 0.38 g paraffin oil was added into the mixing jar; (b) the mixture was mixed at 20 Hz for 60 s using the milling machine, and this was repeated for four times with a 60 s gap between each repeating step (after 3 times' mixing at 20 Hz 60 s, the jar was carefully opened and the residue on the edge is gently swiped into the jar); (c) another 0.35 g graphite powder was added into the jar, and step (b) was repeated..

Alternatively, the carbon paste was obtained from commercial sources (BASi, CF1010, 1 g each bottle). No special preparation was needed for the commercial carbon paste. The carbon paste was ready to use as supplied.

TMB Preparation

TMB for use in a carbon paste working electrode was prepared by the following steps:

(a) 320 mg of TMB was weighed into a 2 mL microcentrifuge tube; (b) the contents of the vial were transferred into a 5 mL grinding jar (1) and the 9 mm grinding ball added afterwards; (c) the jar was place in the milling machine, which was set to 20 Hz with a grinding duration as 60 s; (d) the mixture was ground for 5 times with a 60 s gap between each repeating step (to minimise heat generation during grinding).

PER Preparation

PER for use in a carbon paste working electrode was prepared by the following steps:

(a) 320 mg of TMB was weighed into a 2 mL microcentrifuge tube; (b) the contents of the vial were transferred into a 5 mL grinding jar (1) and the 9 mm grinding ball added afterwards; (c) the jar was place in the milling machine, which was set to 20 Hz with a grinding duration as 60 s: (d) the mixture was ground for 5 times with a 60 s gap between each repeating step (to minimise heat generation during grinding).

Paste Preparation for Working Electrode

A paste for use in the preparation of a carbon paste electrode for use as a first working electrode was prepared by the steps set out below. The carbon paste comprises 5 wt % TMB and 7 wt % PER.

(a) 23.4 mg of ground TMB was weighed into a 2 mL microcentrifuge tube; (b) 32.8 mg of ground PER was weighed into a 2 mL microcentrifuge tube; (c) 412.5 mg of CP was weighed into a 2 mL microcentrifuge tube; (d) the TMB was then added to the CP; (e) the microcentrifuge tube containing the TMB and CP was then shaken vigorously for about 30 s to mix the TMB and CP; (f) the mixture was then placed in a 5 mL grinding jar together with a 9 mm grinding ball; (g) the jar was place in the milling machine, which was set to 20 Hz with a grinding duration as 30 s; (h) the mixture was ground for 6 times with a 60 s gap between each repeating step (to minimise heat generation during grinding); (i) the PER was then added to the mixing jar, and steps (g) and (h) repeated.

During the mixing/milling steps, the jar was opened after every third repetition to mix material at the top end of the jar back in with the other material in the jar.

A paste for use in the preparation of a carbon paste electrode for use as a second working electrode was prepared by steps (a) and (c) to (h) set out above. The carbon paste comprises 5 wt % TMB.

Storage Conditions

The pastes for the preparation of the electrodes was stored in a vacuum desiccator (vacuum level of ca. 900 mbar) with silica gel (Sigma, product number S7625).

The carbon pastes were stored in a sealed amber vial at room temperature.

*Buffer*

A buffer solution for use in the preparation vessel of the invention was prepared. The buffer solution is suitable for preparing a faecal sample for electrochemical analysis, particularly the buffer solution is suitable for allowing haemoglobin in a sample to be detected. Thus, the buffer comprises a cell lysing agent to lyse blood cells.

Buffer A is a citrate-phosphate buffer with added KCl and 0.05 % saponin and 0.05 % ProClin 300, i.e. 1x Buffer A (pH 5.0): 450 mL citrate-phosphate 0.1 M, pH 5.0, 45 mL KCl 1 M, 0.05% saponin, 0.05% ProClin 300.

The buffer was prepared in the following sequence:

(a) 1 M KCL: 37.28 g in 500 ml milliQ water; (b) 1M citrate-phosphate buffer, pH 5.0: 257.5 ml $Na_2HPO_4$ 0.2 M + 242.5 ml citric acid 0.1 M; (c) 0.2 M $Na_2HPO_4$: 14.2 g in 500 ml milliQ water. Note that this can take up to 1 h to dissolve (on a hot plate at 60°C); (d) 0.1 M Citric acid: 9.6 g in 500 ml milliQ water; (e) adding 0.05 % ProClin 300: 0.25 ml in 499.75 ml buffer A; (f) adding 0.05 % saponin: 0.25 g in 500 ml buffer A with ProClin 300. However, normally it is done by adding 2.5 ml of 10 % saponin stock solution into 497.5 ml of buffer A with ProClin 300 because of the difficulty in handling the small amount of saponin; (g) 10% saponin stock solution: 2 g saponin in 20 ml milliQ water

Water used is milliQ water (typically > 15 MΩ•cm).

*Preparation of Screen Printed Electrode*

A patterned substrate of the type shown in Figure 1(a) was prepared by screen printing conducting pastes onto a white polyester sheet (Kemafoil MTSL-Win 350 μm thickness) using pastes from Gwent Electronic Materials Ltd: Carbon paste C2030519P4, Insulator paste D2071120D1, Silver/Silver Chloride Paste 60/40 C2030812D3. The tracks were singulated by laser cutting the cards on a flat bed.

A 2 mm diameter silver/silver chloride electrode was printed over an exposed 1.4 mm width carbon conductor track (the central track seen in Figure 1 (a)). 3 mm diameter first and second working electrodes were printed onto exposed electrical paste (the tracks neighbouring the central track seen in Figure 1(a)).

The electrode diameter of 3 mm was created by depositing working electrode paste through a 3 mm diameter circle created by punching through a piece of "Scotch" tape mask. The hole-punch used: KNIPEX 9070220 with diameter range of holes = 2 to 5 mm, using the 3 mm setting.

The general method of preparation was that described for the preparation of the electrode array shown in Figure 1(f).

The screen printed electrode is provided with an absorbent pad (which is shown as 12 in Figures 1(e) and (f)). The pad was incorporated to block large solid particles from the sample interfering with the sensor and for wicking buffer and sample to the sensor when in a vertical orientation. Pads made from non-woven materials were used. Specification: AF International, brand name: Safecloths, product code: SCH025. Pad size: 14 mm by 14 mm.

The thickness of the carbon paste working electrodes using the above printing method was about 0.2 mm. The required thickness was estimated to be in the range 0.05 mm to 0.2 mm, but was not characterised in detail. The paste was held in position by its inherent viscosity and surface energy and was not cured. This means a rather thick layer compared to standard electrically conductive screen printed paste layers is required. The paste edge quality is important in defining the overall surface area which is preferred to be within a +/ 5% tolerance.

The sensor signal response is proportional to the electrode area and the amount of the analyte (e.g. blood) in the sample, hence consistent area is required for blood measurement accuracy. It is envisaged the paste could be applied by a squeegee and stencil system e.g. as in commercial solder paste and battery paste deposition processes.

The first and second working electrodes prepared from commercial carbon paste were analysed before and after deposition. The data below is based on the measurements on 15 pieces of samples before and after the deposition of paste by the hand stencil method described above.

| Weight (g) | Average | STDEV |
|---|---|---|
| First working electrode paste | 0.0008 g | 20% |
| Amount of paste wasted during the packing of the first working electrode | 0.0007 g | 37% |
| Percentage of paste wasted during the packing of the first working electrode | 52% | 14% |
| Second working electrode paste | 0.001 g | 15% |
| Amount of paste wasted during the packing of the second working electrode | 0.001 g | 14% |
| Percentage of paste wasted during the packing of the second working electrode | 47% | 14% |

The first and second working electrodes prepared from ModeDX carbon paste were analysed before and after deposition. The data below is based on the measurements on 15 pieces of samples before and after the deposition of paste by the hand stencil method described above.

| Weight (g) | Average | STDEV |
|---|---|---|
| First working electrode paste | 0.001 g | 20% |
| Amount of paste wasted during the packing of the first working electrode | 0.001 g | 34% |
| Percentage of paste wasted during the packing of the first working electrode | 53% | 34% |
| Second working electrode paste | 0.001 g | 15% |

The estimation of the paste wasted during the hand deposition of the second working electrode is about 50%, which is comparable to that of the commercial carbon pastes.

*Test Sample*

A test sample for use in testing the electrodes was prepared.

Human Hb powder was obtained from Sigma (cat no H7379-1G). For a standard test, Hb stock solution of 10 mg/mL was used, i.e. 10 mg Hb powder in 1 ml milliQ water (> 15 M$\Omega$cm). The tests were carried out in buffer A and 0.05 mg/ml Hb (i.e. 15 $\mu$L of 10 mg/mL Hb was added into 3 mL to 15$\mu$L buffer A).

*Electrochemistry*

The screen printed electrodes were connected to a power supply and current detector. An Agilent data acquisition system (DAQ) was used.

27

A voltage of + 30 mV was applied between the working and reference electrode with currents flowing between the working and counter electrodes. The working electrode was at ground with the reference electrode at - 30 mV.

The expected current range for a blood detection assay was - 100 nA to + 900 nA.

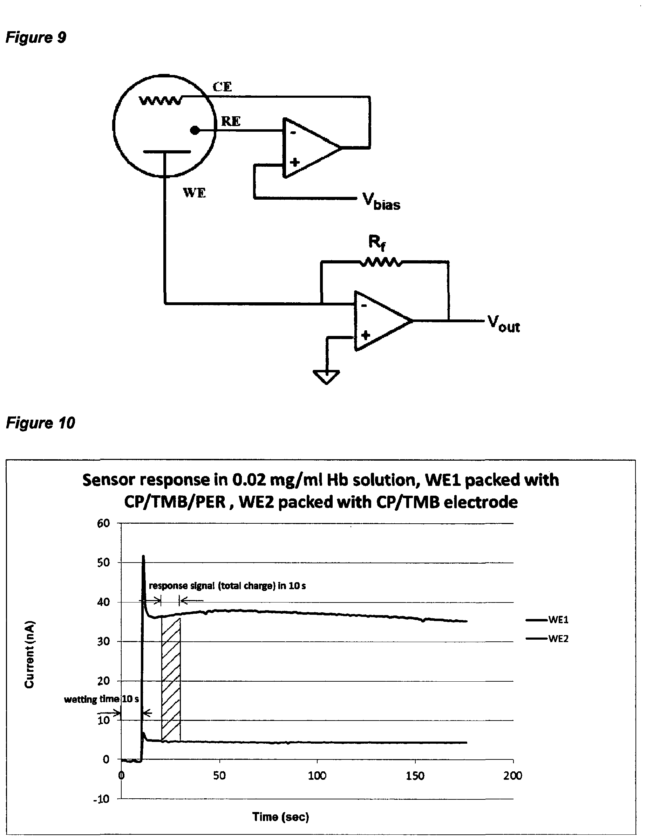

The electrochemical cell control was based on a common three-electrode cell circuit with fixed voltage control (as shown in the schematic of Figure 9). The working electrode (WE) potential is set versus a stable reference electrode (RE) potential and current is allowed to flow between the WE and counter electrode (CE) when immersed in an electrolyte, typically a buffer with salt. When a substance which is electrochemically active is to be analysed and is added to the electrolyte it can be oxidised or reduced at the appropriate applied potential at WE and loses or accepts electrons, thus changing the current at Vout. The amount of electrons transferred due to the substance is proportional to the amount of the substance, which may be blood haemoglobin in the preferred embodiments of the invention.

The present system employs two working electrodes, WE1 (first working electrode) and WE2 (second working electrode), with an additional op-amp for measuring both current outputs (Vout). WE1 is for measuring blood haemoglobin and WE2 is a control background measure used to indicate the sensor has not degraded or affected by interfering substances.

The measurement data acquisition duration is 3 mins with wetting time of 10 s for development. Data analysis is based on the electrochemical response signals recorded at 20-30 s or 1.5-2.5 min for comparison to historical parameters. The timings may refer to the period from the initial contact of the electrodes with the test mixture (which may be the time when a detectable signal is first detected in the electrochemical sensor). This time may be a predicted contact time based on the wetting of the absorbent pad.

*Example Test Method*

The first test method was performed using a test sample prepared from the Hb stock. The method comprises the following steps:

(a) 100 $\mu$L of test solution (buffer A only or buffer A with appropriate amount of Hb stock solution added) was pipetted onto the absorbent pad of the screen printed electrode; (b) the Agilent data acquisition system (DAQ) was started; (c) 30 mV was applied to the working electrodes vs the reference electrode after a wetting time of 10 s; (d) the data was recorded for a further 170 s and the DAQ was stopped and the voltage supply was switched off.

The second test method was performed using a bran stool sample and Hb stock. The method comprises the following steps:

(a) 2.2 g all-bran was weighed into a red-top polypropylene container; (b) 5 ml boiled water was added into the container; (c) the mixture was then soaked for 2 min and stirred evenly after that; (d) for a test with 5% all-bran stool in buffer A solution with 0.05 mg/ml Hb, 0.2 g all bran was weighed into a weighing boat and 20 $\mu$L of 10 mg/mL Hb stock solution was added into all-bran stool and allowed to soak for 1 min. The all-bran and Hb was then gently mixed; (e) the mixture of all-bran with Hb was then transferred into a pre-measured 4 mL to 20 $\mu$L of buffer A to make up a test solution; (f) the sample was analysing by repeating steps (a) to (d) above using the all-bran and Hb sample,

The third test method was performed using a bran stool sample and human whole blood. The method comprises an initial calibration procedure comprising the following steps:

(a) 20 $\mu$L of human whole blood was diluted by 100-fold in 2 mL milliQ water; (b) colorimetric detection based on the 96 well plate, where:

(i) Blank and Calibrator. Pipette 25 $\mu$L water (Blank) and 25 $\mu$L Calibrator into wells of a clear bottom 96-well plate. Transfer 100 $\mu$L water into the blank (column A1-H1) and calibrator wells (column A2-H2). The diluted calibrator was equivalent to 100 mg/dL (i.e. 1 mg/mL) haemoglobin; (ii) 125 $\mu$L of whole blood with 100 fold dilution was added into A3-H3; (iii) incubate 5 min at room temperature. Read OD at 390-405 nm (peak 400 nm); (iv) the Hb in the human whole blood is determined by the colorimetric detection (BioTEK);

The Hb concentration based on the colorimetric detection is calculated by: Subtract blank OD (water) from the Calibrator and Sample OD values. The haemoglobin concentration of sample is calculated as:

$$= \frac{ODSAMPLE - ODBLANK}{ODCALIBRATOR - ODBLANK} \times 100 \times n \, (mg/dL)$$

ODSAMPLE, ODCALIBRATOR and ODBLANK are OD values of the sample, the Calibrator and water. 100 mg/dL is the equivalent haemoglobin concentration of the diluted calibrator. n is the dilution factor (100 for blood samples).

The whole blood stock solution was made according to the determined Hb concentration by adding appropriate amount of milliQ water into it. The test procedures for the bran stool samples described above was repeated with the whole blood sample using the screen printed electrodes of the invention.

*Data Analysis*

Method (1): Calculate the total charge (nC=nA·t, t=1 s) over 60 s, i.e. 1.5 min to 2.5 min.

Method (2): Calculate the total charge (nC=nA·t, t=1 s) over 10 s, i.e. 20 s to 30 s.

A typical sensor raw data curve is shown in Figure 10, with the first 10 s as the sensor wetting time. The response signal in nA was recorded at every second. Response signals from the second working electrode (WE2) were used as background baseline electrode to be subtracted by the actual blood haemoglobin response signals from the first working electrode (WE1), i.e. WE1-WE2. An example for calculating the total charge in 20-30 s (i.e. 10 s in total) is indicated in the graph at the column filled with stripes. The calculation process after the subtraction is as follows:

$$SUM \sum\nolimits_{t=20}^{29} i_t \, (nC) = Current(nA, \, t=20 \, s) \times 1 \, s + Current(nA, \, t=21 \, s) \times 1 \, s + Current(nA, \, t=22 \, s) \times 1 \, s + \cdots + Current(nA, \, t=29 \, s) \times 1 \, s$$

The table below is based on the analysis of three batches of working electrode blends. The repeating tests were carried out on different days. Electrodes were tested in buffer A only, 0.02 mg/mL Hb in buffer A and 0.1 mg/mL Hb in buffer A. The first working electrode (WE1) was packed with a full blend (CP/TMB/PER), the second working electrode (WE2) was packed with a baseline blend (CP/TMB). For each batch of blend, 15 replicates tests were performed under test conditions.

| Hb Con (mg/mL) | WE 1 (60s) nC | WE 1 STDEV (60 s) nC | WE2 (60 s) nC | WE2 STDEV (60 s) nC | WE1-WE2 (60 s) nC | WE1-WE2 STDEV (60 s) nC |
|---|---|---|---|---|---|---|
| 0 | 311 | 18 | 227 | 19 | 77 | 54 |
| 0.02 | 1580 | 346 | 254 | 29 | 1232 | 489 |
| 0.1 | 7107 | 965 | 274 | 25 | 6682 | 1391 |

| Hb Con (mg/mL) | WE 1 (10 s) nC | WE 1 STDEV (10 s) nC | WE2 (10 s) nC | WE2 STDEV (10 s) nC | WE1-WE2 (10 s) nC | WE1-WE2 STDEV (10 s) nC |
|---|---|---|---|---|---|---|
| 0 | 38 | 11 | 31 | 46 | 8 | 42 |
| 0.02 | 297 | 57 | 44 | 6 | 241 | 77 |
| 0.1 | 1364 | 146 | 50 | 6 | 1285 | 242 |

Total charges over 60 s refer to the total charge from 1.5 min to 2.5 min; total charges over 10 s refer to the total charge from 20 s to 30 s.

The table below is based on the tests of a 5% all bran in buffer A, i.e. 0.2 g ready made all-bran stool in 4 mL buffer A with 0.1% saponin. The electrodes were tested in buffer A with all-bran, 0.02 mg/mL Hb in buffer A with all-bran and 0.1 mg/L Hb in buffer A with all-bran. Five replicates were carried out at each test concentration.

| Hb Con (mg/mL) | WE 1 (60 s) nC | WE 1 STDEV (60 s) nC | WE2 (60 s) nC | WE2 STDEV (60 s) nC | WE1-WE2 (60 s) nC | WE1-WE2 STDEV (60 s) nC |
|---|---|---|---|---|---|---|
| 0 | -138 | 483 | 239 | 6 | -126 | 484 |
| 0.02 | 231 | 176 | 67 | 690 | -237 | 830 |
| 0.1 | 2849 | 695 | 290 | 82 | 3466 | 751 |

| Hb Con (mg/mL) | WE 1 (10 s) nC | WE 1 STDEV (10 s) nC | WE2 (10 s) nC | WE2 STDEV (10 s) nC | WE1-WE2 (10 s) nC | WE1-WE2 STDEV (10 s) nC |
|---|---|---|---|---|---|---|
| 0 | -23 | 75 | 35 | 6 | -33 | 77 |
| 0.02 | 12 | 24 | 40 | 0.7 | -36 | 24 |
| 0.1 | 193 | 44 | 46 | 10 | 208 | 52 |

Total charges over 60 s refer to the total charge from 1.5 min to 2.5 min; total charges over 10 s refer to the total charge from 20 s to 30 s.

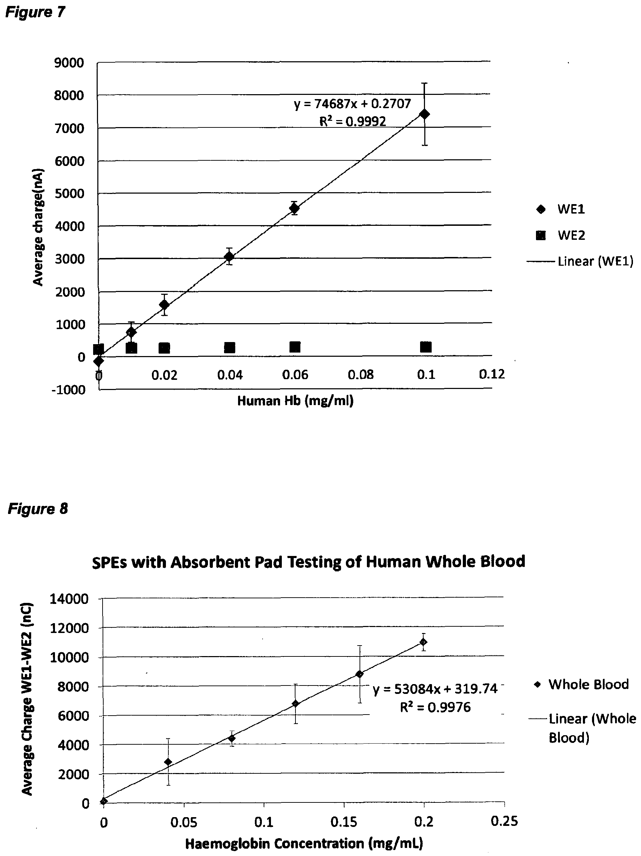

Figures 7 and 8 show the increase in recorded average charge with the increase in Hb concentration. The data is recorded in a similar manner to the data described above, with the current values recorded from 1.5 to 2.5 mins. Figure 8 shows the results for a human whole blood sample as described above, and Figure 7 shows the results for a test sample comprising Hb stock

### Additional Examples

A personal test device of the type shown in Figure 6 was used to determine the haemoglobin content of simulated stool and faecal samples. The results were compared with a commercially available HM-Jack test.

### Tests on Control Samples

A personal test device was prepared having the sampler (64), housing (65), a first part of an analytical unit (70) and a second part of an analytical unit (71) as shown in Figure 6c (and shown also in Figure 6a, 6b and 6c. A total of 200 test devices were prepared.

The first part of the analytical unit (70) was prepared with two working electrodes, where the first working electrode was carbon paste with 5 % 3,3',5,5'-tetramethylbenzidine, and 7% sodium perborate monohydrate and the second working electrode was carbon paste with 5 % 3,3',5,5'-tetramethylbenzidine. The counter electrode and reference electrode were as described for the screen printed electrode described above.

The diameter of the working electrode electrical contact was 2 mm. The applied external voltage (from the second part of an analytical unit (71)) was +30 mV. Tests were carried out at room temperature. Buffer A at pH 5 was provided within the housing (65), contained within the preparation vessel (66-59). The composition of Buffer A is discussed above.

Haemoglobin test solutions were prepared at concentrations indicated in the tables below. As an example, a 5 mg/mL Hb test solution was prepared by adding 1 mL milliQ water to 5 mg Hb. Human Hb powder (Sigma) was used.

The volume of test fluid used is also provided in the table below. The experiment time was typically 30 s.

Tests were carried out in 12 different test solutions including buffer A, with five replicates in each test solution giving a total of 60 tests. See the table below for details. The volume of buffer in each buffer capsule was 1.7 mL.

For the calculation of Hb in stool (mg Hb/g stool):

$$\frac{V_{Hb\,stock} \times C_{Hb\,stock}}{M_{stool}}$$

where, $V_{Hb\ stock}$ (mL) is the volume of Hb stock solution added, $C_{Hb\ stock}$ (mg/mL) is the concentration of Hb stock solution used, and $M_{Stool}$ (g) is the mass of the stool picked up by the sampler.

| Test solution (mg/ml Hb). | Hb stock solution used | Hb stock solution added (μl) | Concentration (mg Hb/g stool) |
|---|---|---|---|
| 0.00 | - | 0.0 | 0.000 |
| 0.01 | 5 mg/ml | 3.4 | 0.034 |
| 0.02 | 5 mg/ml | 6.8 | 0.068 |
| 0.04 | 5 mg/ml | 14.0 | 0.140 |
| 0.06 | 5 mg/ml | 20.0 | 0.200 |
| 0.10 | 20 mg/ml | 8.5 | 0.340 |
| 0.20 | 20 mg/ml | 17.0 | 0.680 |
| 0.30 | 20 mg/ml | 25.0 | 1.000 |
| 0.60 | 40 mg/ml | 25.0 | 2.000 |
| 1.00 | 40 mg/ml | 42.0 | 3.360 |
| 2.00 | 80 mg/ml | 42.0 | 6.720 |
| 5.00 | 200 mg/ml | 42.0 | 16.80 |

An all bran sample was prepared in the following manner:

(1) 13.2 g all bran (dry, Kellogg's, Original) was weighed into a red cap polypropylene container;
(2) 30 mL boiled hot water was added into the all bran and the mixture was stirred well;
(3) the all bran and water mixture was cooled to room temperature for about 30 min and was subsequently used in the stool preparation method.

A simulated stool sample was prepared from the all bran sample and analysed by the following method:

(1) 0.5 g all bran was weighed into a diamond shape weighing boat;
(2) for a stool test with Hb present, an amount of Hb test solution was added into the all bran, and mixed well;
(3) a sampler was used to take up an amount of stool;
(4) the sampler was inserted into the housing containing the preparation vessel and the first part of the analytical unit, thereby to pierce the seals of the vessel, exposing the stool sample to Buffer A, and subsequently exposing the electrodes of the first part of the analytical unit to the resulting mixture. The electrochemistry at the electrode surfaces was controlled by the second part of the analytical unit.

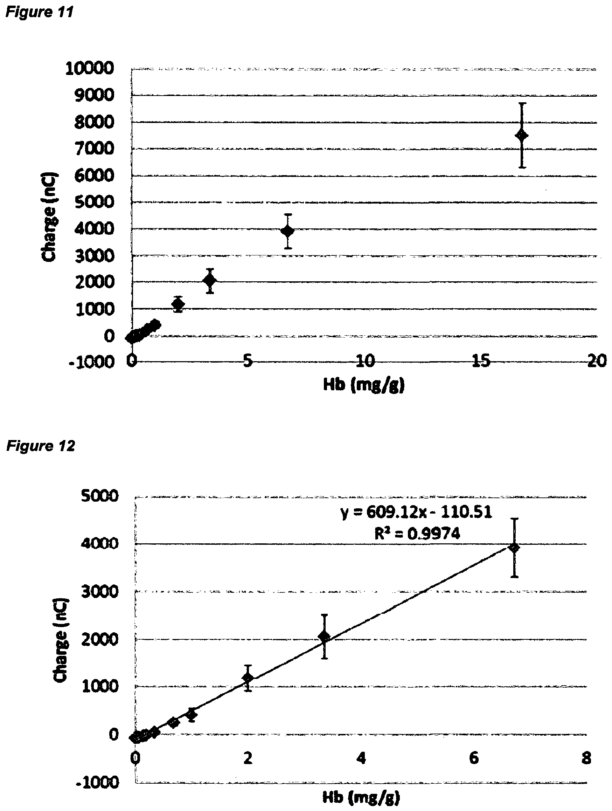

Multiple tests were conducted over three days for stool samples having different Hb concentrations. The combined results, with the calculated standard deviation in replicate experiments, are shown in Figure 11. Figure 12 is a close up of the linear region from Figure 11.

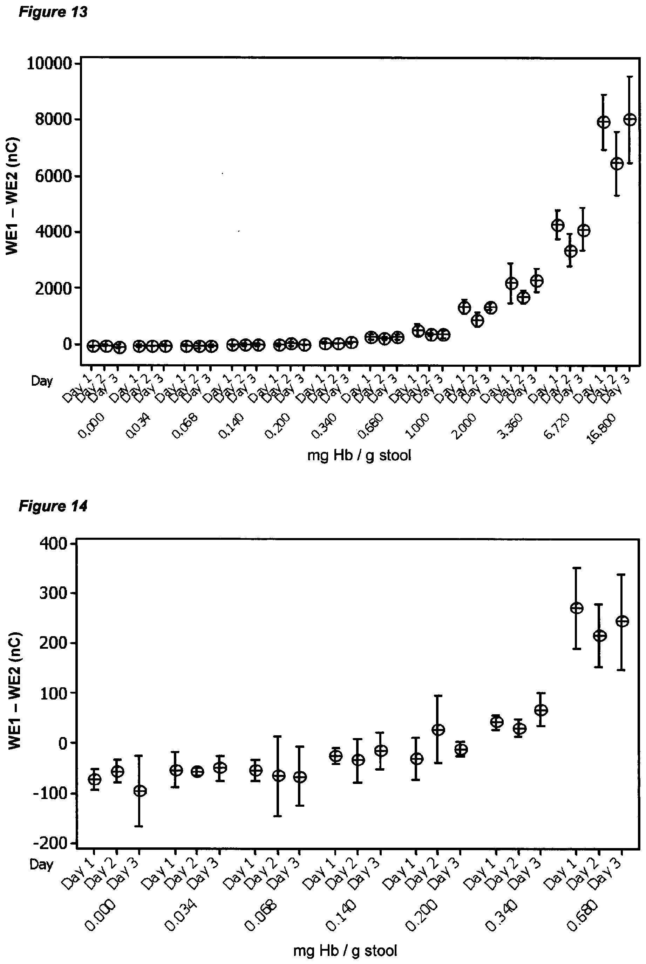

Figure 13 shows the complete study data set with days separated and Figure 14 across the low concentration range, as plots of mean charge (nC) against Hb concentration, with 95% CI error bars shown.

The study over 3 days is as much a test for preparing consistent synthetic faeces Hb controls as it is for assessing the system's measurement variation. The measurements over 3 days were consistent based on the means and overlap of 95% CI's across the Hb range tested.

For this study an acceptable data size was collected since more than 95% of the haemoglobin dose response tests performed reliably, and repeatedly with 95% CI over three days, and therefore the results from the personal test device were deemed to be repeatable and reliable across the required haemoglobin range.

*Tests on Faecal Samples*

The personal test device of Figure 6, as described in the Control Sample experiments above, was used to analyse human faecal samples. The purpose of this study was to perform measurements in a number of real faeces samples from different donors. The intention was that the donors would represent a normal baseline for faeces without blood present, and the samples were verified by performing faecal blood immunoassay tests on each using a commercial available reference system.

In addition, the faecal samples were spiked with known haemoglobin amounts to provide qualitative information on the detection performance with respect to positive blood in faeces results. Cyclic voltammetry scans were also carried out in some of the faecal samples to verify if the correct external potential is applied to the sensor to perform the electrochemical detection in real faeces.

The personal test device included the first part of the analytical unit, which was prepared as described above. As before, the diameter of the working electrode electrical contact was 2 mm. The applied external voltage (from the second part of an analytical unit (71)) was +30 mV. Tests were carried out at room temperature. Buffer A at pH 5 was provided within the housing (65), contained within the preparation vessel (67). The composition of Buffer A is discussed above. The volume of test fluid used for each test was 1.7 mL (this is the volume of buffer A contained within the preparation vessel).

A haemoglobin stock solution was prepared by adding 1 mL milliQ water to 20 mg Hb powder. The volume of Hb stock solution added to a test, sample where appropriate, was 50 μL.

The experiment time was typically 30 s.

Stool samples from 45 donors were supplied by Tissue Solution Ltd, Glasgow, UK. Freshly collected samples were stored for no more than 7 days in a PS container with lid, in a refrigerator (typically at 4°C). The tests were carried out in the Possilpark Health Centre, Glasgow, UK.

The clinic testing procedure was as follows:

(1) the sampler was used to pick up ca. 0.5 g stool from a weighing boat;
(2) where the stool was to be spiked with Hb, 50 μl of 20 mg/mL Hb mixture was added to the surface of the stool sample, otherwise the stool was not treated;
(3) the sampler was inserted into the housing containing the preparation vessel and the first part of the analytical unit, thereby to pierce the seals of the vessel, exposing the stool sample to Buffer A, and subsequently exposing the electrodes of the first part of the analytical unit to the resulting mixture. The electrochemistry at the electrode surfaces was controlled by the second part of the analytical unit.

For each stool sample, six recordings were taken. Three tests were carried out on the raw (untreated) stool sample, and three tests were carried out on stool spiked with Hb. A comparative test was carried out using Hema-screenTM (product code HSSPCAS-10) as a reference assay, for both the raw and spiked stool.

Residues of the stool and Hb were observed in the weighing boat after the sampler was use to pick up stool. Therefore the amount of stool and Hb residue within the tested sample varied from test to test.

Data was analysed for 39 donors and the summary statistics are displayed in the table below:

| Sample | WE Sum 21-30s Charge (nC) | Mean (nC) | SD (nC) |
|--------|---------------------------|-----------|---------|
| raw | WE1 | -103.47 | 75.54 |
| raw | WE 2 | 4.508 | 17.91 |
| Hb | WE1 | 594.4 | 835.47 |
| Hb | WE2 | 8.204 | 15.25 |

The recorded data for six donors was discounted when it was noticed that a second analytical part 71 had become contaminated by a leaking preparation vessel. Hence, the table above refers to 39 donors out of the total of 45 donors.

The test results are summarised in the table below:

| | Hb present | Hb absent | |
|---------|-----------|-----------|---|
| True +ve | 38 | 34 | **Sensitivity:** 97.4359 |
| False -ve | 1 | 5 | **Specificity:** 87.17949 |
| Total | 39 | 39 | |

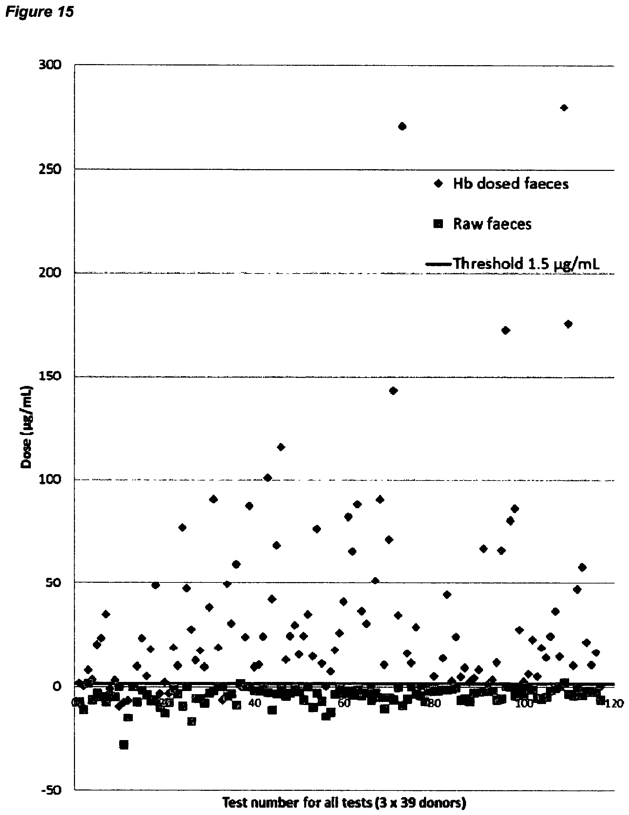

The determined Hb quantities in each tested sample are shown in Figure 15. This is the dose response for 39 donors, where six samples are taken from each stool sample as noted above.

Out of 39 tests on faeces containing added haemoglobin, 38 returned a positive result. Out of 39 tests on faeces with no haemoglobin present (as verified by the reference test) 34 returned a negative result. The reference test provided evidence that the faeces samples did not contain blood, unless otherwise deliberately spiked with Hb. Assuming a detection level at 1.5 µg/mL and a stool volume of 0.5 g and buffer in cartridge volume of 1.7 mL, the calculated detection level of haemoglobin in faeces by weight is ca.5.1 µg/g.

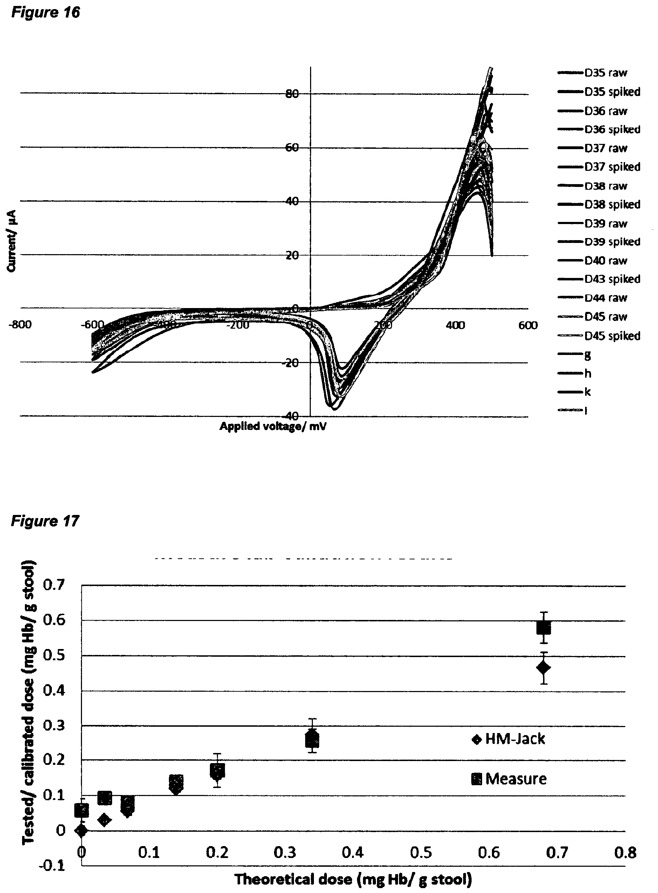

Some of the faecal samples were analysed by cyclic voltammetry, and the voltammetry scans were compared to those recorded against buffer A (g and h), and Buffer A with Hb present (k and l; Hb at 05 mg/mL). The results are shown in Figure 16. The cyclic voltammetry shows the consistency and suitability of the +30 mV measurement voltage determined during development. A carbon paste working electrode containing TMB (5%) and sodium perborate (7%) was used in the electrochemical measurements.

The data collected in this study verified the efficacy of the personal test device in real faeces samples with and without haemoglobin present, with a wide range of dose values which arises with the challenging sample handling and manipulation when spiking in haemoglobin and transferring the sample to the test cartridge. This is acceptable for a qualitative test system.

No significant shifting of the reduction peak potential was observed according to the cyclic voltammetry scans on the faeces, i.e. the applied external potential on working electrode of +30 mV vs Ag/AgCl reference electrode is appropriate for the tests on faeceal samples in the test device.

*Comparison with HM-Jack Test*

The personal test device shown in Figure 6 was compared with a commercial HM-Jack faecal occult blood test, which is an immunoassay-based test. All bran "stool" samples spiked with a series of Hb solutions were analysed using HM-Jack, and the results were compared against the results obtained using the test device of the invention. The results below show that the results from the personal test device and HM-Jack test are well collated. The personal test device is suitable for detecting haemoglobin in the range 0.068 to 0.34 mg Hb/ g stool. This detection range falls usefully within the detection gap between the Guaiac card test (cut-off 0.6 mg Hb/ g stool) and immunochemical tests (cut-off 30 ng/mL, which corresponds to about 0.033 mg Hb/ g stool).

HM-Jack test kits were obtained from Alphalaboratories.

An all bran samples were prepared as described above.

A stool sample was prepared from the all bran sample as described above. A sampler was used to take up an amount of stool and excess stool was removed from the sampler. The residual stool sample on the sampler was tested using the HM-Jack device.

The Hb stock solutions used for the preparation of Hb spiked "stool" is summarised in the table below. All-bran stool samples with and without Hb were prepared. Twelve test solutions with "stool" were prepared. Each all-bran "stool" sample was measure three times.

| Test solution (mg/ml Hb) | Hb stock solution used | Hb stock solution added to 0.5 g "stool "(µl) | Concentration in mg Hb/g all bran |
|---|---|---|---|
| 0 (control) | - | 0 | 0 |
| 0.01 | 5 mg/ml | 3.4 | 0.034 |
| 0.02 | 5 mg/ml | 6.8 | 0.068 |
| 0.04 | 5 mg/ml | 14 | 0.14 |
| 0.06 | 5 mg/ml | 20 | 0.2 |
| 0.1 | 20 mg/ml | 8.5 | 0.34 |
| 0.2 | 20 mg/ml | 17 | 0.68 |
| 0.3 | 20 mg/ml | 25 | 1 |
| 0.6 | 40 mg/ml | 25 | 2 |
| 1 | 40 mg/ml | 42 | 3.36 |
| 2 | 80 mg/ml | 42 | 6.72 |
| 5 | 200 mg/ml | 42 | 16.8 |

The Hb test solution used was prepared e.g. for 5 mg/mL Hb, from 5 mg Hb in 1 mL milliQ water. The Hb was human Hb powder from Sigma.

The test results from the HM-Jack analysis is set out below.

| Theoretical Hb Concentration in Stool (mg Hb/g stool) | HM-Jack Hb Concentration in Stool (mg Hb/ g stool) | Standard Deviation in HM-Jack Concentrations | HM-Jack Average Recorded Signals (ng/mL) |
|---|---|---|---|
| 0 | 0.0002641 | 0.01344 | 0.4 |
| 0.034 | 0.03079 | 0.003631 | 38.37 |
| 0.068 | 0.05575 | 0.01278 | 64.4 |
| 0.14 | 0.1192 | 0.009502 | 140.8 |
| 0.2 | 0.1579 | 0.01041 | 205.2 |
| 0.34 | 0.2724 | 0.04887 | 327.9 |
| 0.68 | 0.466 | 0.04595 | 610.6 |
| 1 | 0.5766 | 0.0926 | 689.9 |
| 2 | 0.5218 | 0.01368 | 669.5 |
| 3.36 | 0.4047 | 0.04861 | 615.9 |
| 6.72 | 0.45 | 0.03487 | 538 |
| 16.8 | 0.3566 | 0.03002 | 479.9 |

Similar stool samples were tested using the device of Figure 6, as described in detail above, and the results are set out below:

| Theoretical Hb Concentration in Stool (mg Hb/g stool) | Average Signal Over 10 s (nC) | Standard Deviation in Average Signal | Calibrated Hb Concentration in Stool (mg Hb/g stool) | Standard Deviation in Calibrated Hb Concentration |
|---|---|---|---|---|
| 0 | -75.67 | 37.45 | 0.0572 | 0.03292 |
| 0.034 | -54.15 | 19.41 | 0.09252 | 0.004702 |
| 0.068 | -63.11 | 44.47 | 0.07781 | 0.01123 |
| 0.14 | -25.52 | 27.21 | 0.1395 | 0.01642 |
| 0.2 | -5.778 | 42.85 | 0.1719 | 0.0478 |
| 0.34 | 45.96 | 23.48 | 0.2569 | 0.03264 |
| 0.68 | 243.6 | 64.7 | 0.5813 | 0.0454 |
| 1 | 412 | 131.3 | 0.8579 | 0.1472 |
| 2 | 1186 | 272.5 | 2.129 | 0.4311 |
| 3.36 | 2059 | 272.5 | 3.561 | 0.5259 |
| 6.72 | 3920 | 622 | 6.617 | 0.8207 |
| 16.8 | 7498 | 1193 | 12.49 | 1.457 |

The theoretical and determined Hb concentrations from the HM-Jack and personal test device experiments are shown in Figure 17.

*References*

EP 1,167,968
JP 06-201701
US 5,658,531
WO 99/38996
WO 2006/085087
WO 2010/129727

**Claims**

1. A personal test device comprising a preparation vessel, a sampler and an analytical unit, wherein:

   (i) the preparation vessel has a first chamber with first and second openings, wherein each of the first and second openings is sealed thereby to seal the first chamber, and the first chamber holds one or more reagents;
   (ii) the sampler is for holding a sample, and the sampler is adapted to pierce the seals at the first and second openings;
   (iii) the analytical unit is adapted to analyse material from the first chamber,

   and the analytical unit comprises an electrochemical sensor.

2. The personal test device according to claim 1, wherein the sampler is for holding a faecal sample and the personal test device is for the analysis of a faecal sample.

3. The personal test device of claim 1 or claim 2, wherein the preparation vessel and/or the analytical unit comprises a first reagent and/or a second reagent, the second reagent being an oxidising agent or a precursor thereof for the first reagent, wherein a reaction between the first reagent and the second reagent is catalysable by an analyte in the sample, such as a protein, to provide a detectable signal.

4. The personal test device of claim 3, wherein the first and second reagents are provided in the analytical unit.

5. The personal test device according to any one of the preceding claims, wherein the analytical unit comprises a first analytical part and a second analytical part adapted to communicate with the first analytical part.

6. The personal test device of claim 3, wherein the first analytical part is integrated with the preparation vessel.

7. The personal test device according to any one of claims 1 to 4, wherein the analytical unit is an electrochemical sensor having a working electrode, a counter electrode, optionally a reference electrode, a power supply and a controller, and optionally the electrochemical sensor is provided with a second working electrode.

8. The personal test device according to claim 7, wherein the working electrode and counter electrode are connectable to a power supply and a controller, and the electrodes are provided in a first part analytical part and the power supply and the controller are provided in a second analytical part, which is connectable to the first part and is electrically communicable with the first analytical part,
   and (i) optionally the first analytical part is integrated with the preparation vessel; and/or (ii) the second analytical part is optionally connectable to and separable from the first analytical part.

9. The personal test device of any one of claims 7 to 8, wherein the electrochemical sensor comprises an electrically conductive porous matrix working electrode.

10. The personal test device of claim 9, wherein the working electrode is an electrically conductive carbon- or graphite-containing porous matrix working electrode, such as wherein the working electrode is a carbon paste electrode.

11. The personal test device of any one of claims 7 to 10, wherein the working electrode contains a first reagent and a second reagent, the second reagent being an oxidising agent or a precursor thereof for the first reagent, wherein a reaction between the first reagent and the second reagent is catalysable by an analyte in the sample, such as a protein, to provide a detectable signal,

and (i) the first reagent is optionally selected from tetramethylbenzidine, alpha guaiaconic acid, 2,2'-azino-bis(3-ethylbenzothiazolidine-6-sulphonic acid), hydroquinone, phenylenediamine, o-dianisidine, o-tolidine (dimethylbenzidine), 6-methoxyquinoline, and 3,3'-diaminobenzidine, and 3-amino-9-ethylcarbazole; and/or (ii) the second reagent is optionally selected from urea peroxide, a perborate compound and a periodate compound.

12. The personal test device of any one of the previous claims, wherein:

(i) the first and second openings of the preparation vessel are opposed; and/or
(ii) the first opening is tapered toward the chamber; and/or.
(iii) the preparation vessel is provided with a second chamber, and the second chamber is connected to the chamber via the second opening, and optionally the analytical unit is adapted to analyse material in the second chamber; and/or
(iv) the sampler has a shaft with a piercing tip at one end, and the shaft is provided with one or more protrusions and/or recesses, and the one or more protrusions are optionally flexible or inflexible.

13. The personal test device of claim 12, wherein the sampler is provided with a seal on the shaft which is suitable for sealing the first opening, wherein the protrusions are provided on the shaft between the piercing tip and the seal.

14. A method for analysing a sample, such as to detect the presence of haemoglobin in the sample, the method comprising the steps of:

(i) providing a personal test device according to any one of claims 1 to 13;
(ii) obtaining a sample using the sampler of the test device;
(iii) piercing the seal at the first opening of the preparation vessel of the test device with the sampler, and permitting at least part of the sampler to enter the first chamber, thereby to expose sample held by the sampler to the one or more reagents in the first chamber;
(iv) subsequently piercing the seal at the second opening of the preparation vessel with the sampler, thereby to permit material in the first chamber to exit the chamber, such as through the second opening;
(v) permitting the analytical unit of the test device to analyse the material from the first chamber.

15. The method of claim 14, wherein the method is for detecting the presence of a blood in a sample, such as a faecal sample.

**Patentansprüche**

1. Persönliche Testvorrichtung, umfassend ein Präparationsgefäß, einen Probennehmer und eine Analyseeinheit, worin:

(i) das Präparationsgefäß eine erste Kammer mit einer ersten und einer zweiten Öffnung hat, worin jede der ersten und zweiten Öffnungen abgedichtet ist, um dadurch die erste Kammer abzudichten, und die erste Kammer ein oder mehrere Reagenzien aufnimmt;
(ii) der Probennehmer zum Aufnehmen einer Probe bestimmt ist und der Probennehmer dafür eingerichtet ist, die Dichtungen an der ersten und zweiten Öffnung zu durchstechen;
(iii) die Analyseeinheit dafür eingerichtet ist, Material aus der ersten Kammer zu analysieren, und die Analyseeinheit einen elektrochemischen Sensor umfasst.

2. Persönliche Testvorrichtung nach Anspruch 1, worin der Probennehmer zum Aufnehmen einer Stuhlprobe bestimmt ist und die Analyseeinheit zur Analyse einer Stuhlprobe bestimmt ist.

3. Persönliche Testvorrichtung nach Anspruch 1 oder Anspruch 2, worin das Präparationsgefäß und/oder die Analyseeinheit ein erstes Reagens und/oder ein zweites Reagens umfasst, wobei das zweite Reagens ein Oxidationsmittel oder eine Vorläufersubstanz davon für das erste Reagens ist, worin eine Reaktion zwischen dem ersten Reagens und dem zweiten Reagens durch einen Analyt in der Probe, wie etwa ein Protein, katalysierbar ist, um ein detektierbares Signal bereitzustellen.

4. Persönliche Testvorrichtung nach Anspruch 3, worin das erste und zweite Reagenz in der Analyseeinheit bereitgestellt werden.

5. Persönliche Testvorrichtung nach einem der vorhergehenden Ansprüche, worin die Analyseeinheit einen ersten Analyseteil und einen zweiten Analyseteil, der dafür eingerichtet ist, mit dem ersten Analyseteil zu kommunizieren, umfasst.

6. Persönliche Testvorrichtung nach Anspruch 3, worin der erste Analyseteil in das Präparationsgefäß integriert ist.

7. Persönliche Testvorrichtung nach einem der Ansprüche 1 bis 4, worin die Analyseeinheit ein elektrochemischer Sensor mit einer Arbeitselektrode, einer Gegenelektrode, optional einer Bezugselektrode, einer Stromversorgung und einer Steuerungseinrichtung ist und worin der elektrochemische Sensor optional mit einer zweiten Arbeitselektrode versehen ist.

8. Persönliche Testvorrichtung nach Anspruch 7, worin die Arbeitselektrode und die Gegenelektrode mit einer Stromversorgung und einer Steuerungseinrichtung verbindbar sind und die Elektroden in einem ersten Analyseteil bereitgestellt sind und die Stromversorgung und die Steuerungseinrichtung in einem zweiten Analyseteil bereitgestellt sind, der mit dem ersten Teil verbindbar ist und mit dem ersten Analyseteil in elektrische Kommunikation gebracht werden kann,
und (i) der erste Analyseteil optional in das Präparationsgefäß integriert ist; und/oder
(ii) der zweite Analyseteil optional mit dem ersten Analyseteil verbindbar und davon trennbar ist.

9. Persönliche Testvorrichtung nach einem der Ansprüche 7 bis 8, worin der elektrochemische Sensor eine Arbeitselektrode aus einer elektrisch leitfähigen porösen Matrix umfasst.

10. Persönliche Testvorrichtung nach Anspruch 9, worin die Arbeitselektrode eine Arbeitselektrode aus einer elektrisch leitfähigen kohlenstoff- oder graphithaltigen porösen Matrix ist, wie etwa worin die Arbeitselektrode eine Kohlenstoffpaste-Elektrode ist.

11. Persönliche Testvorrichtung nach einem der Ansprüche 7 bis 10, worin die Arbeitselektrode ein erstes Reagens und ein zweites Reagens enthält, wobei das zweite Reagens ein Oxidationsmittel oder eine Vorläufersubstanz davon für das erste Reagens ist, worin eine Reaktion zwischen dem ersten Reagens und dem zweiten Reagens durch einen Analyt in der Probe, wie etwa ein Protein, katalysierbar ist, um ein detektierbares Signal bereitzustellen, und (i) das erste Reagens optional aus Tetramethylbenzidin, $\alpha$-Guajakonsäure, 2,2'-Azino-bis(3-Ethylbenzothiazolidin-6-Sulfonsäure), Hydrochinon, Phenylendiamin, o-Dianisidin, o-Tolidin (Dimethylbenzidin), 6-Methoxychinolin, 3,3'-Diaminobenzidin und 3-Amino-9-Ethylcarbazol ausgewählt wird; und/oder (ii) das zweite Reagens optional aus Carbamidperoxid, einer Perboratverbindung und einer Periodatverbindung ausgewählt wird.

12. Persönliche Testvorrichtung nach einem der vorhergehenden Ansprüche, worin:

(i) die erste und zweite Öffnung des Präparationsgefäßes einander gegenüberliegen; und/oder
(ii) die erste Öffnung zur Kammer hin verengt ist; und/oder
(iii) das Präparationsgefäß mit einer zweiten Kammer versehen ist und die zweite Kammer über die zweite Öffnung mit der ersten Kammer verbunden ist und die Analyseeinheit optional dafür eingerichtet ist, Material in der zweiten Kammer zu analysieren; und/oder
(iv) der Probennehmer einen Schaft mit einer stechenden Spitze an einem Ende hat und der Schaft mit einem oder mehreren Vorsprüngen oder Vertiefungen versehen ist und der eine oder die mehreren Vorsprünge optional biegsam oder unbiegsam sind.

13. Persönliche Testvorrichtung nach Anspruch 12, worin der Probennehmer mit einer Dichtung am Schaft versehen ist, die zum Abdichten der ersten Öffnung geeignet ist, worin die Vorsprünge am Schaft zwischen der stechenden Spitze und der Dichtung ausgebildet sind.

14. Verfahren zum Analysieren einer Probe, wie etwa um das Vorhandensein von Hämoglobin in der Probe zu detektieren, wobei das Verfahren die folgenden Schritte umfasst:

(i) Bereitstellen einer persönlichen Testvorrichtung nach einem der Ansprüche 1 bis 13;
(ii) Erlangen einer Probe unter Verwendung des Probennehmers der Testvorrichtung;

(iii) Durchstechen der Dichtung an der ersten Öffnung des Präparationsgefäßes der Testvorrichtung mit dem Probennehmer und Ermöglichen, dass zumindest ein Teil des Probennehmers in die erste Kammer eintritt, um dadurch die durch den Probennehmer aufgenommene Probe einem oder mehreren Reagenzien in der ersten Kammer auszusetzen;

(iv) anschließend Durchstechen der Dichtung an der zweiten Öffnung des Präparationsgefäßes mit dem Probennehmer, um dadurch zu ermöglichen, dass Material in der ersten Kammer die Kammer verlässt, wie etwa durch die zweite Öffnung;

(v) Ermöglichen, dass die Analyseeinheit der Testvorrichtung das Material aus der ersten Kammer analysiert.

**15.** Verfahren nach Anspruch 14, worin das Verfahren zum Ermitteln des Vorhandenseins von Blut in einer Probe, wie etwa einer Stuhlprobe, bestimmt ist.

**Revendications**

**1.** Dispositif de test personnel comprenant un récipient de préparation, un échantillonneur et une unité analytique, dans lequel :

(i) le récipient de préparation a une première chambre avec des première et deuxième ouvertures, dans lequel chacune des première et deuxième ouvertures est scellée hermétiquement pour ainsi sceller hermétiquement la première chambre, et la première chambre contient un ou plusieurs réactifs ;

(ii) l'échantillonneur est destiné à contenir un échantillon, et l'échantillonneur est adapté pour percer les joints au niveau des première et deuxième ouvertures ;

(iii) l'unité analytique est adaptée pour analyser une substance en provenance de la première chambre, et l'unité analytique comprend un capteur électrochimique.

**2.** Dispositif de test personnel selon la revendication 1, dans lequel l'échantillonneur est destiné à contenir un échantillon fécal et le dispositif de test personnel est destiné à l'analyse de l'échantillon fécal.

**3.** Dispositif de test personnel selon la revendication 1 ou la revendication 2, dans lequel le récipient de préparation et/ou l'unité analytique comprennent un premier réactif et/ou un deuxième réactif, le deuxième réactif étant un agent oxydant ou un précurseur de celui-ci pour le premier réactif, dans lequel une réaction entre le premier réactif et le deuxième réactif est catalysable par un analyte dans l'échantillon, tel qu'une protéine, pour fournir un signal détectable.

**4.** Dispositif de test personnel selon la revendication 3, dans lequel les premier et deuxième réactifs sont prévus dans l'unité analytique.

**5.** Dispositif de test personnel selon l'une quelconque des revendications précédentes, dans lequel l'unité analytique comprend une première partie analytique et une deuxième partie analytique adaptée pour communiquer avec la première partie analytique.

**6.** Dispositif de test personnel selon la revendication 3, dans lequel la première partie analytique est intégrée avec le récipient de préparation.

**7.** Dispositif de test personnel selon l'une quelconque des revendications 1 à 4, dans lequel l'unité analytique est un capteur électrochimique ayant une électrode de travail, une contre-électrode, facultativement une électrode de référence, une alimentation électrique et un contrôleur, et facultativement le capteur électrochimique est doté d'une deuxième électrode de travail.

**8.** Dispositif de test personnel selon la revendication 7, dans lequel l'électrode de travail et la contre-électrode peuvent être raccordées à une alimentation électrique et à un contrôleur, et les électrodes sont prévues dans une première partie analytique et l'alimentation électrique et le contrôleur sont prévus dans une deuxième partie analytique, qui peut être raccordée à la première partie et peut communiquer électriquement avec la première partie analytique, et (i) facultativement la première partie analytique est intégrée dans le récipient de préparation ;

et/ou (ii) la deuxième partie analytique peut facultativement être raccordée à et séparée de la première partie analytique.

9. Dispositif de test personnel selon l'une quelconque des revendications 7 à 8, dans lequel le capteur électrochimique comprend une électrode de travail à matrice poreuse électriquement conductrice.

10. Dispositif de test personnel selon la revendication 9, dans lequel l'électrode de travail est une électrode de travail à matrice poreuse contenant du carbone ou du graphite électriquement conductrice, tel que dans lequel l'électrode de travail est une électrode en pâte de carbone.

11. Dispositif de test personnel selon l'une quelconque des revendications 7 à 10, dans lequel l'électrode de travail contient un premier réactif et un deuxième réactif, le deuxième réactif étant un réactif oxydant ou un précurseur de celui-ci pour le premier réactif, dans lequel une réaction entre le premier réactif et le deuxième réactif est catalysable par un analyte dans l'échantillon, tel qu'une protéine, pour fournir un signal détectable,
et (i) le premier réactif est facultativement sélectionné parmi la tétraméthylbenzidine, l'acide alpha-guaiaconique, l'acide 2,2'-azino-bis(3-éthylbenzothiazolidine-6-sulfonique), l'hydroquinone, la phénylènediamine, l'o-dianisidine, l'o-tolidine (diméthylbenzidine), la 6-méthoxyquinoline et la 3,3'-diaminobenzidine et le 3-amino-9-éthylcarbazole ;
et/ou (ii) le deuxième réactif est facultativement sélectionné parmi le peroxyde d'urée, un composé de perborate et un composé de périodate.

12. Dispositif de test personnel selon l'une quelconque des revendications précédentes, dans lequel :

(i) les première et deuxième ouvertures du récipient de préparation sont opposées ; et/ou
(ii) la première ouverture rétrécit progressivement vers la chambre ; et/ou
(iii) le récipient de préparation est doté d'une deuxième chambre, et la deuxième chambre est raccordée à la chambre via la deuxième ouverture, et facultativement l'unité analytique est adaptée pour analyser une substance dans la deuxième chambre ; et/ou
(iv) l'échantillonneur a un axe avec une pointe de perçage au niveau d'une extrémité, et l'axe est doté d'une ou plusieurs saillies et/ou évidements, et les une ou plusieurs saillies sont facultativement souples ou raides.

13. Dispositif de test personnel selon la revendication 12, dans lequel l'échantillonneur est doté d'un joint sur l'axe qui est adéquat pour sceller hermétiquement la première ouverture, dans lequel les saillies sont prévues sur l'axe entre la pointe de perçage et le joint.

14. Procédé destiné à analyser un échantillon, tel que pour détecter la présence d'hémoglobine dans l'échantillon, le procédé comprenant les étapes consistant à :

(i) prévoir un dispositif de test personnel selon l'une quelconque des revendications 1 à 13 ;
(ii) obtenir un échantillon en utilisant l'échantillonneur du dispositif de test ;
(iii) percer le joint au niveau de la première ouverture du récipient de préparation du dispositif de test avec l'échantillonneur, et permettre qu'au moins une partie de l'échantillonneur entre dans la première chambre, pour ainsi exposer l'échantillon contenu par l'échantillonneur à l'un ou aux plusieurs réactifs dans la première chambre ;
(iv) percer par la suite le joint au niveau de la deuxième ouverture du récipient de préparation avec l'échantillonneur, pour ainsi permettre qu'une substance dans la première chambre sorte de la chambre, tel qu'à travers la deuxième ouverture ;
(v) permettre que l'unité analytique du dispositif de test analyse la substance en provenance de la première chambre.

15. Procédé selon la revendication 14, dans lequel le procédé est destiné à détecter la présence de sang dans un échantillon, tel qu'un échantillon fécal.

Figure 1

a             b             c

d             e             f

**Figure 2**

**Figure 3**

**Figure 4**

*Figure 5*

Figure 6a

60

Figure 6b

61

62

63

**Figure 6c**

**Figure 6d**

**Figure 7**

**Figure 8**

SPEs with Absorbent Pad Testing of Human Whole Blood

*Figure 9*

*Figure 10*

Figure 11

Figure 12

*Figure 13*

*Figure 14*

Figure 15

*Figure 16*

*Figure 17*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006085087 A
- JP 6201701 A
- US 5658531 A
- WO 9938996 A
- WO 2010129727 A
- EP 1167968 A
- WO 2009055306 A